(19) Europäisches Patentamt
European Patent Office
Office européen des brevets

(11) Numéro de publication: **0 071 535**
**B1**

(12) # FASCICULE DE BREVET EUROPÉEN

(45) Date de publication du fascicule du brevet: **14.01.87**

(51) Int. Cl.⁴: **C 07 D 333/24**, C 07 D 409/12, A 61 K 31/38

(21) Numéro de dépôt: **82401411.2**

(22) Date de dépôt: **29.07.82**

(54) **2- ou/et 3-substitué 4-(thiényl acétamido) phénoxy hydroxy propylamines, leur préparation, leur utilisation en thérapeutique et nouveaux intermédiaires.**

(30) Priorité: **29.07.81 FR 8114773**

(43) Date de publication de la demande:
**09.02.83 Bulletin 83/6**

(45) Mention de la délivrance du brevet:
**14.01.87 Bulletin 87/3**

(84) Etats contractants désignés:
**AT BE CH DE GB IT LI LU NL SE**

(56) Documents cité:
**DE-A-2 024 001**
**DE-A-2 636 725**
**DE-A-2 720 613**
**FR-A-2 458 548**
**FR-A-2 464 952**

**Le dossier contient des informations techniques présentées postérieurement au dépôt de la demande et ne figurant pas dans le présent fascicule.**

(73) Titulaire: **CARPIBEM (CENTRE D'ACTIVITE ET DE RECHERCHE PHARMACEUTIQUE INDUSTRIELLE BIOLOGIQUE ET MEDICALE) Société Anonyme dite:, 128, rue Danton, F-92500 Rueil- Malmaison (FR)**

(72) Inventeur: **Teulon, Jean- Marie, 12, Résidence du Bel Ebat 56, avenue de Verdun, F-78170 La Celle-Saint- Cloud (FR)**
Inventeur: **Bouley, Etienne Marie Marcel, 9, rue Moguez, F-92210 Saint- Cloud (FR)**

(74) Mandataire: **Combe, André, CABINET BEAU DE LOMENIE 55 rue d'Amsterdam, F-75008 Paris (FR)**

EP 0 071 535 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne, en tant que produits industriels nouveaux, les dérivés de la phénoxy hydroxy propylamine de formule générale (I) et leurs sels d'addition d'acides. Elle concerne également le procédé de préparation desdits produits et leur application en thérapeutique. Elle concerne en outre les composés intermédiaires nouveaux permettant la synthèse desdits produits.

Le brevet français 2455545 décrit en tant que produits des anilides d'acides furanne-2-carboxyliques substitués et les propriétés β-bloquantes de ces produits. Les essais comparatifs fournis dans ce brevet montrent que le produit de l'exemple 1 dudit brevet présente, par rapport au propanol et au practolol, une activité supérieure de blocage des β-récepteurs; la toxicité inférieure dudit produit de l'exemple (par rapport aux composés de référence) confère audit produit des avantages supplémentaires. On notera toutefois que l'activité antihypertensive eventuelle dudit produit n'est ni décrite ni revendiquée dans ledit brevet.

La présente invention concerne l'ensemble formé par:

a) les produits de formule générale:

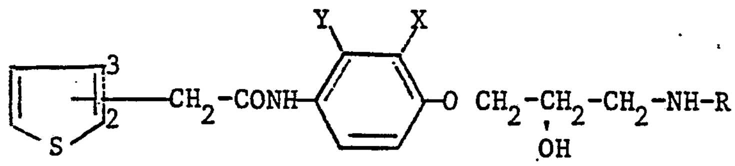

(I)

dans laquelle

- la fonction acétamide peut être en position -2 ou -3 sur l'heterocycle thiophène,
- X représente un atome d'halogène, un groupement allylique ou méthoxy ou un groupement nitrile et peut être l'hydrogène dans le cas seulement où Y est un halogène,
- Y représente généralement l'hydrogène mais peut être un halogène,
- R représente un radical alkyle inférieur en $C_1$-$C_6$ linéaire ou ramifié,

et b) leurs sels d'addition d'acides.

Les composés de formule (I) selon l'invention sont synthétisés par action d'une base $NH_2R$, R étant défini comme cidessus, sur un composé de formule (II) sans solvant ou dans un solvant organique usuel principalement les alcools à une température comprise entre 20 et 150° C.

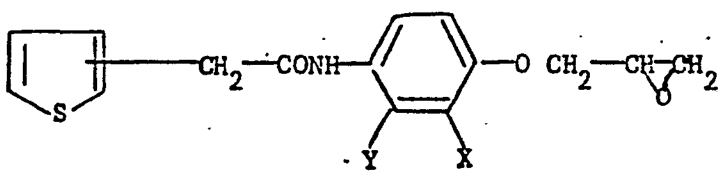

(II)

Dans la formule (II), X et Y sont définis comme ci-dessus

D'une façon générale les composés de formule (II) peuvent être obtenus par réaction d'un phénol de formule (III) avec une épihalogénhydrine par exemple 1 épichlorhydrine ou 1 épibromhydrine, le phénol de formule (III) étant préalablement métallé par des agents usuels de métallation tels que la soude la potasse un méthylate et un éthylate en milieu hydroalcoolique ou alcoolique à température ambiante. Toutefois, ces procédés connus donnent de faibles rendements et des produits de qualité médiocre.

C'est pourquoi la demanderesse a découvert et mis au point un procédé consistant à faire réagir le phénol de formule (III) avec un excès d'épichlorhydrine en présence d'un catalyseur tel que le benzyl triméthyl ammonium chlorure à une température de 110 à 130° C; ce procédé donne de très bons rendements et des produits cristallisés et parfaitement définis.

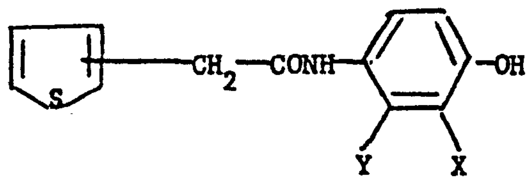

(III)

Dans la formule (III) X et Y sont définis comme ci-dessus.

Les composés de formule (III) sont synthétisés par action du chlorure de l'acide thiényl acétique correspondant sur des amino phénols de formule (IV) soit en présence du double d'amino phénol soit en présence d'une base telle que par exemple la triéthylamine dans un solvant usuel comme l'acétone.

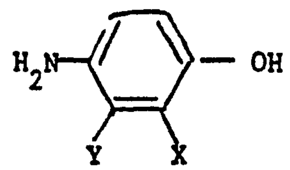

(IV)

Dans la formule (IV), X et Y sont définis comme ci-dessus.

Les sels d'addition d'acides des composés de formule (I) peuvent être obtenus par réaction avec un acide minéral ou organique selon une méthode connue en soi. Parmi les acides utilisables à cet effet, on peut notamment citer les acides chlorhydrique, bromhydrique, sulfurique, phosphorique, oxalique succinique, méthsne sulfonique, cyclohexyl sulfamique, formique aspartique glutamique N-acétyl aspartique, N-acétyl glutamique ascorbique maléique malique, fumarique, lactique benzoique cinnamique, 4-toluène sulfonique.

Selon l'invention, les nouveaux composés sont doués d'activités pharmacologiques intéressantes et peuvent être utiles en thérapeutique comme agent bloqueur cardiosélectif bêta-adrénergique et anti-hypertenseur. On propose donc des compositions thérapeutiques utiles notamment pour le traitement des affections cardiovasculaires caractérisées en ce qu'elles renferment en association avec un excipient physiologiquement acceptable au moins un composé de formule (1) ou l'un de ses sels d'addition d'acides non toxiques.

L'invention concerne em outre les produits intermédiaires nouveaux.

D'autres caractéristiques et avantages de l'invention seront mieux compris à la lecture qui va suivre de quelques exemples de préparation mullement limitatifs mais donnés à titre d'illustration. Le tableau 1 ci-après donne la formule développée de certains composés exemplifiés.

**Exemple 1**

2-cyano 4-(2-thiényl acétamido) phénol.

Formule (III) X = CN acétamide en 2

A une solution de 27,3 gde 2-cyano 4-amino phénol dans 350 ml d'acétone on ajoute goutte à goutte sous bonne agitation une solution de 16,4 g du chlorure de l'acide 2-thiényl acétique dans 50 ml d'acétone. Après la fin de l'addition le mélange réactionnel est agité encore durant 4 heures puis laissé au repos une nuit. Le chlorhydrate de 2-cyano 4-amino phénol qui a précipité est essoré, soigneusement lavé à l'acétone puis le filtrat acétonique est concentré sous vide. Le résidu obtenu est repris par de l'eau glacée additionnée d'acide chlorhydrique normal sous bonne agitation. Les cristaux formés sont essorés, soigneusement lavés à l'eau puis à l'éther et séchés. On obtient ainsi 19 g de 2-cyano 4-(2-thiényl acétamido) phénol sous forme de cristaux de point de fusion 150° C.

**Exemple 2**

2-cyano 4-(3-thiényl acétamido) phénol.

Formule (III) X = CN, acétamide en 3

Selon le mode opératoire de l'exemple 1 mais en utilisant 27,3 g de 2-cyano 4-amino phénol et 16,4 g du chlorure de l'acide 3-thiényl acétique on obtient 26 g de 2-cyano 4-(3-thiényl acétamido) phénol sous forme de cristaux de point de fusion 105-108° C.

**Exemple 3**

<u>2-allyl 4-(2-thiényl acétamido) phénol.</u>
Formule (III) X = $CH_2$-CH = $CH_2$, acétamide en 2
Selon le mode opératoire de l'exemple 1 mais en utilisant 32,5 g de 2-allyl 4-amino phénol et 17,5 g du chlorure de l'acide 2-thiényl acétique on obtient 16,2 g de 2-allyl 4-(2-thiényl acétamido) phénol sous forme de cristaux de point de fusion 106-109° C.

**Exemple 4**

<u>3-,[2-cyano 4-(2-thiényl acétamido) phénoxyl] 1,2-époxy propane.</u>
Formule (II) X = CN, acétamide en 2
On porte sous agitation à 110° C un mélange de 19 g de 2-cyano 4-(2-thiényl acétamido) phénol préparé à l'exemple 1 et de 70 ml d'épichlorhydrine. On ajoute ensuite au mélange 1,5 g de benzyltriméthyl ammonium chlorure et ls réaction est portée au reflux durant 30 min. Le mélange réactionnel est ensuite refroidi autour de 60° C et additionné d'eau à 60° C. Le produit cristallise alors. Les cristaux sont essorés, soigneusement lavés à l'éther et séchés. On récupère ainsi 14 g de 3-[2-cyano 4-(2-thiényl acétamido) phénoxy] 1,2-époxy propane sous forme de cristaux de point de fusion 119-121° C.

**Exemple 5**

<u>3-[2-cyano 4-(3-thiényl acétamido) phénoxyl 1,2-époxy propane.</u>
Formule (II) X = CN, acétamide en 3
Selon le mode opératoire de l'exemple 4 mais en utilisant 26 g de 2-cyano 4-(3-thiényl acétamido) phénol préparé à l'exemple 2 on obtient 23 g de 3-[2-cyano 4-(3-thiényl acétamido) phénoxy] 1,2-époxy propane sous forme de cristaux de point de fusion 148-150° C.

**Exemple 6**

<u>3-[2-allyl 4-(2-thiényl acétamido) phénoxy] 1,2-époxy propane.</u>
Formule (11) X = $CH_2$-CH = $CH_2$, acétamide en 2
Selon le mode opératoire de l'exemple 4 mais en utilisant 13,2 g de 2-allyl 4-(2-thiényl acétamido) phénol préparé à l'exemple 3 on obtient 11,5 g de 3- [2-allyl 4-(2-thiényl acétamido) phénoxy] 1,2-époxy propane sous forme de cristaux de point de fusion 90-95° C.

**Exemple 7**

<u>Chlorhydrate de 1-[2-cyano 4-(2-thiényl acétamido) phénoxy] 3-terbutylamino 2-propanol.</u>

Formule (I) R = 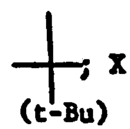 ; X = CN ; acétamide en 2
(t-Bu)

On porte au reflux durant 5 h une solution de 8 g de 3-[2-cyano 4-(2 thiényl acétamido) phénoxy] 1,2-époxy propane préparé à l'exemple 4 et de 10 ml de terbutylamine dans 50 ml de méthanol. Le mélange réactionnel est ensuite concentré sous vide repris par un mélange d'eau et de glace et acidifié par de l'acide acétique. Les produits neutres sont extraits à l'éther et la phase aqueuse est alcalinisée à froid par une solution de soude normale. Après extraction su chloroforme que l'on sèche sur carbonate de sodium filtre et évapore, on récupère un résidu qui est dissous dans l'isopropanol. Par addition d'éther chlorhydrique jusqu'à pH acide à cette solution isopropanolique, on observe la formation de cristsux qu'on laisse développer et qu'on essore lave soigneusement à l'éther et qu'on sèche. On récupère ainsi 5,3 g du chlorhydrate de 1-[2-cyano 4-(2-thiényl acétamido) phénoxy] 3-terbutylamino 2-propanol sous forme de cristaux de point de fusion 174-177° C.

**Exemple 8**

1-12-cyano 4-(2-thiényl acétamido) phénoxy] 3-isopropylamino 2-pro-panol (base et chlorhydrate).

Formule (I) R = 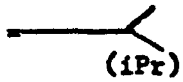 ; X = CN; acétamide en 2

(iPr)

On porte au reflux durant 5 h une solution de 4 g de 3-[2-cysno 4-(2-thiényl acétamido) phénoxy] 1 2-époxy propane préparé à l'exemple 4 et de 10 ml d'isopropylamine dans 50 ml de méthanol. Le mélange réactionnel est ensuite concentré sous vide, repris par un mélange d'eau et de glace et scidifié par de l'acide acétique. Les produits neutres sont extraits à l'éther et la phase aqueuse est alcslinisée à froid par une solution de soude normale. Après extraction au chloroforme que l'on sèche sur carbonate de sodium filtre et évspore, on récupère un résidu qui cristallise dans l'éther Les cristaux sont essorés et lavés à l'éther puis séchés. On récupére ainsi 3 g de 1-[2-cyano 4-(2-thiényl acétamido) phénoxy] 3-isopropylamino 2-propanol sous forme de cristaux de point de fusion 122° C.

Chlorhydrate.

Ces 3 g de cristaux sont dissous dans l'acétone et additionnés d'éther chlorhydrique jusqu'à pH acide. Les cristaux formés sont essorés, lavés à l'acétone puis à l'éther et séchés. On obtient 2 3 g de chlorhydrate de 1-[2-cyano 4-(2-thiényl acétamldo) phénoxy] 3-isopropylamino 2-propanol sous forme de cristaux de point de fusion 152-153° C.

**Exemple 9**

Chlorhydrate de 1-[2-cyano 4-(3-thiényl acétamido) phénoxy] 3-terbutylamino 2-propanol.

Formule (I) R = 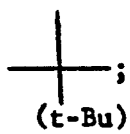 ; X = CN ; acétamide en 3

(t-Bu)

Selon le mode opératoire de l'exemple 7 mais en utilisant 8 g de 3-[2-cyano 4-(3-thiényl acétamido) phénoxy] 1,2-époxy propane préparé à l'exemple 5 et 10 ml de terbutylamine on récupère 5,2 g du chlorhydrate de 1-[2-cyano 4-(3-thiényl acétamido) phénoxy] 3-terbutylamino 2-propanol sous forme de cristaux de point de fusion 177-180° C.

**Exemple 10**

Chlorhydrate de 1-'2-cyano 4-(3-thiényl acétamido) phénoxyl 3-isopropylamino 2-propanol.

Formule (I) R = 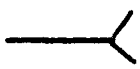 ; X = CN ; acétamide en 3

(iPr)

Selon le mode opératoire de l'exemple 7 mais en utilisant 7,2 g de 3-[2-cyano 4-(3-thiényl acétamido) phénoxy] 1,2-époxy propane préparé à l'exemple 5 et 10 ml d'isopropylamine on récupère 5,4 g du chlorhydrate de 1-[2-cyano 4-(3-thiényl acétamido) phénoxy] 3-isopropylamino 2-propanol sous forme de cristaux de point de fusion 164-167° C.

**Exemple 11**

1-[2-allyl 4-(2-thiényl acétamido) phénoxy] 3-terbut-ylamino 2-propanol.

Formule (I) R = 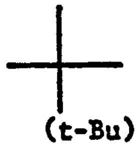 ; X = $CH_2-CH=CH_2$ ; acétamide en 2

(t-Bu)

Selon le mode opératoire de l'exemple 8 mais en utilisant 11 g de 3-[2-allyl 4-(2-thiényl acétamido) phénoxy] 1,2-époxy propane préparé à l'exemple 6 et 15 ml de terbutylamino on obtient 9 g de 1-[2-allyl 4-(2-thiényl acétamido) phénoxy] 3-ter-butylamino 2-propanol sous forme de cristaux de point de fusion 92-94° C.

**Exemple 12** 2-allyl 4-(3-thiényl acétamido) phénol

Formule (III) X = $CH_2$- CH = $CH_2$ Y = H acétamide en 3

A une solution de 25 g de 2-allyl 4-amino phénol dans 250 ml d'acétone on ajoute goutte à goutte sous bonne agitation une solution de 13 4 g du chlorure de l'acide 3-thiényl acétique dans 50 ml d'acétone. Après la fin de l'addition le mélange réactionnel est agité encore durant 4 heures puis laissé au repos une nuit. Le mélange réactionnel est ensuite concentré sous vide puis le résidu repris par un mélange eau-glace est extrait à l'éther. La phase éthérée est ensuite extraite à froid par une solution normale de potasse. La solution aqueuse basique est alors acidifiée à froid par une solution d'acide chlorhydrique normale et le produit organique qui précipite alors est extrait à l'éther qu'on sèche sur sulfate de sodium filtre et évapore. On obtient ainsi 12,4 g de 2-allyl 4-(3-thiényl acétamido) phénol sous forme de cristaux de point de fusion 100° C.

**Exemple 13** 2-bromo 4-(2-thiényl acétamido) phénol

Formule (III) X = Br Y = H acétamide en 2

A une solution de 24,8 g de 2 bromo 4-aminophénol dans 250 ml d'acétone on ajoute goutte à goutte sous bonne agitation une solurion de 10,5 g du chlorure de l'acide 2-thiényl acétique dans 25 ml d'acétone. Après la fin de l'addition le mélange réactionnel est agité encore durant 4 heures puis laissé au repos une nuit. Le chlorhydrate de 2-bromo 4-amino phénol qui a précipité est essoré, soigneusement lavé à l'acétone puis le filtrat acétonique est concentré sous vide. Le résidu obtenu est repris par de l'eau glacée en milieu légèrement acide par addition d'une solution d'acide chlorhydrique normale sous bonne agitation. Les cristaux formés sont essorés, soigneusement lavés à l'eau puis à l'éther isopropylique et séchés. On obtient ainsi 18 g de 2-bromo 4-(2-thiényl acétamido) phénol sous forme de cristaux de point de fusion 114-116° C.

**Exemple 14** 2-bromo 4-(3-thiényl acétamido) phénol

Formule (III) X = Br Y = H acétamide en 3

Selon le mode opératoire de l'exemple 13 mais en utilisant 26 g de 2-bromo 4-aminophénol et 11,1 g du chlorure de l'acide 3-thiényl acétique on obtient 21 g de 2-bromo 4-(3-thiényl acétamido) phénol sous forme de cristaux de point de fusion 136° C.

**Exemple 15** 2-chloro 4-(2-thiényl acétamido) phénol

Formule (III) X = Cl Y = H acétamide en 2

Selon le mode opératoire de l'exemple 13 mais en utilisant 30 g de 2-chloro 4-amino phénol et 16,8 g du chlorure de l'acide 2-thiényl acétique on obtient 23 6 g de 2-chloro 4-(2-thiényl acétamido) phénol sous forme de cristaux de point de fusion 122-4° C.

**Exemple 16** 2-chloro 4-(3-thiényl acétamido) phénol

Formule (III) X = Cl Y = H acétamide en 3

Selon le mode opératoire de l'exemple 13 mais en utilisant 27,5 g de 2-chloro 4-amino phénol et 15,4 g du chlorure de l'acide 3-thiényl acétique on obtient 22,5 g de 2-chloro 4-(3-thiényl acétamido) phénol sous forme de cristaux de point de fusion 120° C.

**Exemple 17** 2-fluoro 4-(2-thiényl acétamido) phénol

Formule (II) X = F Y = H acétamide en 2

Selon le mode opératoire de l'exemple 13 mais en utilisant 21 g de 2-fluoro 4-aminophénol et 13,3 g du chlorure de l'acide 2-thiényl acétique on obtient 21,5 g de 2-fluoro 4-(2-thiényl acétamido) phénol sous forme de cristaux de point de fusion 138-140° C.

**Exemple 18** 2-fluoro 4-(3-thiényl acétamido) phénol

Formule (III) X = F Y = H acétamide en 3

Selon le mode opératoire de l'exemple 13 mais en utilisant 21 g de 2-fluoro 4-amino phénol et 13 3 g du chlorure de l'acide 3-thiényl acétique on obtient 20 g de 2-fluoro 4-(3-thiényl acétamido) phénol sous forme de cristaux de point de fusion 138-140° C.

**Exemple 19** 2-méthoxy 4-(2-thiényl acétamido) phénol

Formule (III) X = $OCH_3$ Y = H acétamide en 2

Selon le mode opératoire de l'exemple 13 mais en utilisant 32,5 g de 2-méthoxy 4-amino phénol et 18,8 g du chlorure de l'acide 2-thiényl acétique on obtient 25,5 g de 2-méthoxy 4-(2-thiényl acétamido) phénol sous forme de cristaux de point de fusion 137° C.

**Exemple 20** 2-méthoxy 4-(3-thiényl acétamido) phénol

Formule (III) X = $OCH_3$ Y = H acétamide en 3

Selon le mode opératoire de l'exemple 13 mais en utilisant 23,4 g de 2-méthoxy 4-amino phénol et 13,5 g du chlorure de l'acide 3-thiényl acétique on obtient 23 g de 2-méthoxy 4-(3-thiényl acétamido) phénol sous forme de cristaux de point de fusion 160° C.

**Exemple 21** 2,3-dichloro 4-(2-thiényl acétamido) phénol

Formule (III) X = Y = Cl acétamide en 2

Selon le mode opératoire de l'exemple 13 mais en utilisant 27,5 g de 2,3-dichloro 4-amino phénol et 12,4 g du chlorure de l'acide 2-thiényl acétique on obtient 22,3 g de 2,3-dichloro 4-(2-thiényl acétamido) phénol sous forme de cristaux de point de fusion 152° C.

**Exemple 22** 2,3-dichloro 4-(3-thiényl acétamido) phénol

Formule (III) X = Y = Cl acétamide en 3

Selon le mode opératoire de l'exemple 13 mais en utilisant 30 g de 2 3-dichloro 4-aminophénol et 13,5 g du chlorure de l'acide 3-thiényl acétique on obtient 25 g de 2,3-dichloro-4-(3-thiényl acétamido) phénol sous forme de cristaux de point de fusion 170° C.

**Exemple 23** 3-chloro 4-(2-thiényl acétamido) phénol

Formule (III) X = H Y = Cl acétamide en 2

Selon le mode opératoire de l'exemple 13 mais en utilisant 26,5 g de 3-chloro 4-amino phénol et 14,8 g du chlorure de l'acide 2-thiényl acétique on obtient 22,5 g de 3-chloro 4-(2-thiényl acétamido) phénol sous forme de cristaux de point de fusion 134° C.

**Exemple 24** 3-chloro 4-(3-thiényl acétamido) phénol

Formule (III) X = H Y = Cl acétamide en 3

Selon le mode opératoire de l'exemple 13 mais en utilisant 30 g de 3-chloro 4-amino phénol et 16,8 g du chlorure de l'acide 3-thiényl acétique on obtient 24 g de 3-chloro 4-(3-thiényl acétamido) phénol sous forme de cristaux de point de fusion 131° C.

**Exemple 25** 3-[2-allyl 4-(3-thiényl acétamido) phénoxy]

1,2-époxy propane.

Formule (II) X = $CH_2$-CH = $CH_2$ Y = H acétamide en 3

On porte sous agitation à 110° C un mélange de 12 g de 2-allyl 4-(3-thiényl acétamido) phénol préparé à l'exemple 12 et de 30 ml d'épichlorhydrique. On ajoute ensuite au mélange 1 g de benzyl triméthyl ammonium chlorure et la réaction est portée au reflux durant 30 minutes. Le mélange réactionnel est ensuite refroidi autour de 60° C et additionné d'eau à 60° C. Après agitation durant 30 minutes le produit cristallise. Les cristaux sont essorés soigneusement lavés à l'éther et séchés. On récupère ainsi 8,3 g de 3-[2-allyl 4-(3-thiényl acétamido) phénoxy] 1,2-époxy propane sous forme de cristaux de point de fusion 87-89° C.

**Exemple 26** 3-[2-bromo 4-(2-thiényl acétamido) phénoxy]

1,2-époxy propane

Formule (II) X = Br Y = H acétamide en 2

Selon le mode opératoire de l'exemple 25 mais en utilisant 18 g de 2-bromo 4-(2-thiényl acétamido) phénol préparé à l'exemple 13 on obtient 15 g de 3-[2-bromo 4-(2-thiényl acétamido) phénoxy] 1,2-époxy propane sous forme de cristsux de point de fusion 124-127° C.

**Exemple 27** 3-[2-bromo 4-(3-thiényl acétamido) phénoxy]

1,2-époxy propane

Formule (II) X = Br Y = H acétamide en 3

Selon le mode opératoire de l'exemple 25 mais en utilisant 21 g de 2-bromo 4-(3-thiényl acétamido) phénol préparé à l'exemple 14 on obtient 17 g de 3-[2-bromo 4-(3-thiényl acétamido) phénoxy] 1,2-époxy propane sous forme de cristaux de point de fusion 128° C.

**Exemple 28** 3-[2-chloro 4-(2-thiényl acétamido) phénoxr]

1,2-époxy propane

Formule (II) X = Cl Y = H acétamide en 2

Selon le mode opératoire de l'exemple 25 mais en utilisant 23,6 g de 2-chloro 4-(2-thiényl acétamido) phénol préparé à l'exemple 15 on obtient 20 g de 3-[2-chloro 4-(2-thiényl acétamido) phénoxy] 1,2-époxy propane sous forme de cristaux de point de fusion 103-105° C.

**Exemple 29** 3-[2-chloro 4-(3-thiényl acétamido) phénoxy]

1,2-époxy propane

Formule (II) X = Cl Y = H acétamide en 3

Selon le mode opératoire de l'exemple 25 mais en utilisant 27,5 g de 2-chloro 4-(3-thiényl acétamido) phénol préparé à l'exemple 16 on obtient 22,5 g de 3-[2-chloro 4-(3-thiényl acétamido) phénoxy] 1,2-époxy propane sous forme de cristaux de point de fusion 120° C.

**Exemple 30** 3-[2-fluoro 4-(2-thiényl acétamido)phénoxy]

1,2-époxy propane

Formule (II) X = F Y = H acétamide en 2

Selon le mode opératoire de l'exemple 25 mais en utilisant 21,5 g 2-fluoro 4-(2-thiényl acétamido) phénol préparé à l'exemple 17 on obtient 24 g de 3-[2-fluoro 4-(2-thiényl acétamido) phénoxy] 1,2-époxy propane sous forme de cristaux de point de fusion 86° C.

**Exemple 31** 3-[2-fluoro 4-(3-thiényl acétamido) phénoxy]

1,2-époxy propane.

Formule (II) X = F Y = H acétamide en 3

Selon le mode opératoire de l'exemple 25 mais en utilisant 20 g de 2-fluoro 4-(3-thiényl acétamido)phénol préparé à l'exemple 18 on obtient 18,4 g de 3-[2-fluoro 4-(3-thiényl acétamido)phénoxy] 1,2-époxy propane sous forme de cristaux de point de fusion 120° C.

**Exemple 32** 3-[2-méthoxy 4-(2-thiényl acétamido)phénoxy]

1,2-é-poxy propane

Formule (II) X = $OCH_3$ Y = H acétamide en 2

Selon le mode opératoire de l'exemple 25 mais en utilisant 25,5 g de 2-méthoxy 4-(2-thiényl acétamido) phénol préparé à l'exemple 19 on obtient 20 g de 3-[2-méthoxy 4-(2-thiényl acétamido) phénoxy] 1,2-époxy propane sous forme de cristaux de point de fusion 135° C.

**Exemple 33** 3-[2-méthoxy 4-(3-thiényl acétamido) phénoxy]

1,2-époxy propane

Formule (II) X = $OCH_3$ Y = H acétamide en 3

Selon le mode opératoire de l'exemple 25 mais en utilisant 23 g de 2-méthoxy 4-(3-thiényl acétamido) phénol préparé à l'exemple 20 on obtient 22,5 g de 3-[2-méthoxy 4-(3-thiényl acétamido) phénoxy] 1,2-époxy propane sous forme de cristaux de point de fusion 161-164° C.

**Exemple 34** 3-[2,3-dichloro 4-(2-thiényl acétamido) phénoxy]

1,2-époxy propane

Formule (II) X = Y = Cl acétamide en 2

Selon le mode opératoire de l'exemple 25 mais en utilisant 22,3 g de 2,3-dichloro 4-(2-thiényl acétamido) phénol préparé à l'exemple 21 on obtient 24 g de 3-[2,3-dichloro 4-(2-thiényl acétamido) phénoxy] 1,2-époxy propane sous forme de cristaux de point de fusion 85° C.

**Exemple 35** 3-[2 3-dichloro 4-(3-thiényl acétamido) phénoxy]

1,2-époxy propane

Formule (II) X = Y = Cl acétamide en 3

Selon le mode opératoire de l'exemple 25 mais en utilisant 25 g de 2,3-dichloro 4-(3-thiényl acétamido) phénol préparé à l'exemple 22 on obtient 24,5 g de 3-[2,3-dichloro 4-(3-thiényl acétamido) phénoxy] 1,2-époxy propane sous forme de cristaux de point de fusion 103-105° C.

**Exemple 36** 3-[3-chloro 4(2-thiényl acétamido) phénoxy]

1,2-époxy propane

Formule (II) X = H Y = Cl acétamide en 2

Selon le mode opératoire de l'exemple 25 mais en utilisant 22,5 g de 3-chloro 4-(2-thiényl acétamido) phénol préparé à l'exemple 23 on obtient 19,2 g de 3-[3-chloro 4-(2-thiényl acétamido) phénoxy] 1 2-époxy propane sous forme de cristaux de point de fusion 92° C.

**Exemple 37** 3-[3-chloro 4-(3-thiényl acétamido) phénoxyl

1,2-époxy propane

Formule (II) X = H Y = Cl acétamide en 3

Selon le mode opératoire de l'exemple 25 mais en utilisant 24 g de 3-chloro 4-(3-thiényl acétemido) phénol préparé à l'exemple 24 on obtient 29 g de 3-[3-chloro 4-(3-thiényl acétamido)phénoxy] 1,2-époxy propane sous forme de cristsux de point ds fusion 75° C.

**Exemple 38** 1-[ 2-allyl 4-(3-thiényl acétamido) phénoxy]

3-terbutylamino 2-propanol (base et chlorhydrate)

Formule (I) R = terbutyls X = $CH_2$- CH=$CH_2$ Y = H acétamide en 3

On porte au reflux durant 6 heures une solution de 10 g de 3-[2-allyl 4-(3-thiényl acétamido) phénoxy] 1 2-époxy propane préparé à l'exemple 25 et de 10 ml de terbutylamine dans 50 ml de méthanol. Le mélange réactionnel est ensuite concentré sous vide, repris par un mélange d'eau et de glace et acidifié par de l'acide scétique. Les produits neutres sont extraits à l'éther et la phase aqueuse est alcalinisée à froid par une solution de soude normale. Après extraction au chloroforme que l'on sèche sur carbonate de sodium filtre et évapore, on récupère un résidu qui cristallise dans l'éther. Les cristaux sont essorés et lavés à l'éther puis séchés. On récupère ainsi 6,8 g de 1-[2-allyl 4-(3-thiényl acétamido) phénoxy] 3-terbutylamino 2-propanol sous forme de cristaux de point de fusion 91-93° C.

Chlorhydrate

Ces 6,8 g de cristaux sont dissous dans l'isopropsnol et additionnés d'éther chlorhydrique jusqu'à pH acide. Les cristaux formés sont essorés lavés avec un peu d'isopropanol et d'éther et séchés. On obtient 6 g de chlorhydrate de 1-[2-allyl 4-(3-thiényl acétamido) phénoxy] 3-terbutylamino 2-propanol sous forme de cristaux de point de fusion 163-166° C.

**Exemple 39** 1-[2-bromo 4-(2-thiényl acétamido) phénoxy]

3-terbutylamino 2-propanol (base et maléate).

Formule (I) R = terbutyle X = Br Y = H acétamide en 2

Selon le mode opératoire de l'exemple 38 mais en utilisant 10 g de 3-[2-bromo 4-(2-thiényl acétamido) phénoxy] 1,2-époxy propane préparé à l'exemple 26 et 10 ml de terbutylamine on récupère 9 g de 1-[2-bromo 4-(2-thiényl acétamido) phénoxy] 3-terbutylamino 2-propanol sous forme de cristaux de point de fusion 105° C.

Maléate

Ces 9 g de base sont dissous dans l'acétate d'éthyle et additionnés d'une quantité stoéchiométrique d'acide maléique dissous dsns l'acétate d'éthyle. Les cristaux formés sont essorés lavés à l'acétate d'éthyle et à l'éther puis séchés. On récupère ainsi 10 2 g du maléate de 1-[2-bromo 4-(2-thiényl acétamido)phénoxy] 3-terbutylamino 2-propanol sous forme de cristaux de point de fusion 172-5° C.

**Exemple 40** 1-[2-bromo 4-(2-thiényl acétamido) phénoxy)

3-isopropylamino 2-propanol (base et chlorhydrate)

Formule (I) R = isopropyle X = Br Y = H acétamide en 2

Selon le mode opératoire de l'exemple 38 mais en utilisant 5 g de 3-[2-bromo 4-(2-thiényl acétamido) phénoxy] 1,2-époxy propane préparé à l'exemple 26 et 10 ml d'isopropylamine on récupère 4,2 g de 1-[2-bromo 4-(2-thiényl acétamido) phénoxy] 3-isopropyl-amino 2-propanol sous forme de cristaux de point de fusion 150° C.

>Chlorhydrate

Ces 4,2 g de cristaux sont dissous dans l'acétone et additionnés d'éther chlorhydrique jusqu'à pH acide. Les

cristaux formés sont essorés, lavés avec un peu d'acétone et d'éther et séchés. On obtient 3 6 g de chlorhydrate de 1-[2-bromo 4-(2-thiényl acétamido) phénoxy] 3-isopropylamino 2-propanol sous forme de cristaux de point de fusion 177-160° C.

**Exemple 41** 1-[2 bromo 4-(3-thiényl acétamido) phénoxy]

3-terbutylamino 2-propanol (chlorhydrate)

Formule (I) R = terbutyle X = Br Y = H acétamide en 3

Selon le mode opératoire de l'exemple 38 mais en utilisant 10 g de 3-[2-bromo 4-(3-thiényl acétamido) phénoxy] 1,2-époxy propane préparé à l'exemple 27 et 10 ml de terbutylamine on récupère 9,5 g de 1-[2-bromo 4-(3-thiényl scétamido) phénoxy] 3-terbutyla-mino 2-propanol sous forme d'une huile.

Cette huile est solubilisée dans l'acétone et additionnée d'éther chlorhydrique jusqu'à pH acide.Les cristaux qui se forment alors sont essorés, lavés avec um peu d'acétate et d'éther et séchés. On obtient 8,9 g de chlorhydrate de 1-[2-bromo 4-(3-thiényl acétamido) phénoxy] 3-terbutylamino 2-propanol sous forme de cristaux de point de fusion 197-199° C.

**Exemple 42** 1-[2-bromo 4-(3-thiényl acétamido) phénoxy]

3-Isorapylamino 2-propanol (base et chlorhydrate).

Formule (I) R = isopropyle X = Br Y = H acétamide en 3

Selon le mode opératoire de l'exemple 38 mais en utilisant 7 g de 3-[2-bromo 4-(3-thiényl acétamido)phénoxy] 1,2-époxy propane préparé à l'exemple 27 et 10 ml d'isopropylamine on récupère 5,8 g de 1-[2-bromo 4-(3-thiényl acétamido) phénoxy] 3-isopropylamino 2-propanol sous forme de cristaux de point de fusion 124-127° C.

Chlorhydrate

Ces 5,8 g de cristaux sont dissous dans du méthanol et additionnés d'éther chlorhydrique jusqu'à pH acide. Après addition d'un peu d'éther les cristaux formés sont essorés lavés avec un peu de méthanol et d'éther et séchés.

On obtient 5 g de chlorhydrate de 1-[2-bromo 4-(3-thiényl acétamido) phénoxy] 3-isopropylamino 2-propanol sous forme de cristaux de point de fusion 168-171° C.

**Exemple 43** 1-[2-chloro 4-(2-thiényl acétamido phénoxy]

3-terbutylamino 2-propanol (base et maléate)

Formule (I) R = terbutyle X = Cl Y = H acétamide en 2

Selon le mode opératoire de l'exemple 38 mais en utilisant 10 g de 3-[2-chloro 4-(2-thiényl acétamido) phénoxy] 1,2-époxy propane préparé à l'exemple 28 et 10 ml de terbutylamine om récupère 9,5 g de 1-[2-chloro 4-(2-thiényl acétamido) phénoxy] 3-terbu-tylamino 2-propanol sous forme de cristaux de point de fusion 129° C.

Maléate

Ces 9,5 g de base sont dissous dans l'acétone et additionnés de 2,8 g d'acide maléique en solution dans l'acétone. Les cristaux formés sont essorés, lavés à l'acétone et à l'éther puis séchés. On récupère ainsi 9,8 g de maléate de 1-[2-chloro 4-(2-thiényl acétamido) phénoxy] 3-terbutylamino 2-propanol sous forme de cristaux de point de fusion 180-183° C.

**Exemple 44** 1-[2-chloro 4-(2-thiényl acétamido) phénoxy]

3-isopropylamino 2-propanol (base et maléate)

Formule (I) R = isopropyle X = Cl Y = H acétamide en 2

Selon le mode opératoire de l'exemple 38 mais en utilisant 10 g de 3-[2-chloro 4-(2-thiényl acétamido) phénoxy] 1,2-époxy propane prépsré à l'exemple 28 et 15 ml d'isopropylamine on récupère 8 g de 1-[2-chloro 4-(2-thiényl acétamido) phénoxy] 3-isopro-pylamino 2-propanol sous forme de cristaux de point de fusion 147° C.

Maléate

Ces 8 g de base sont dissous dans l'acétone et additionnés de la quantité stoéchiométrique d'acide maléique en solution dans l'acétone. Les cristaux formés sont essorés lavés à l'acétone et à l'éther puis séchés. On récupère ainsi 9,7 g de maléste de 1-[2-chloro 4-(2-thiényl acétamido) phénoxy] 3-isopropylamino 2-propanol sous forme de cristaux de point de fusion 137-139° C.

**Exemple 45** 1-[2-chloro 4-(3-thiényl acétamido) phénoxy)

3-terbutylamino 2-propanol (base et chlorhydrate)

Formule (I) R = terbutyle X = Cl Y = H acétamide en 3

Selon le mode opératoire de l'exemple 38 mais en utilisant 10 g de 3-[2-chloro 4-(3-thiényl acétamido) phénoxy] 1,2-époxy propane préparé à l'exemple 29 et 10 ml de terbutylamine on récupère 9,5 g de 1-[2-chloro 4-(3-thiényl acétamido) phénoxy] 3-terbutylamino 2-propanol sous forme de cristaux de point de fusion 109° C.

Chlorhydrate

Ces 9,5 g de cristaux sont dissous dans l'acétone et additionnés d'éther chlorhydrique jusqu'à pH acide. Les cristaux formés sont essorés, lavés avec um peu d'acétone et d'éther et séchés. On obtient 9,8 g de chlorhydrate de 1-[2-chloro 4-(3-thiényl acétamido) phénoxy] 3-terbutylamino 2-propanol sous forme de cristaux de point ds fusion 194-196° C.

**Exemple 46** 1-[2-chloro 4-(3-thiényl acétamido) phénoxy]

3-isopropylamino 2-propanol (base et chlorhydrate)

Formule (1) R = isopropyle X = Cl Y = H acétamide en 3

Selon le mode opératoire de l'exemple 38 mais en utilisant 8,2 g de 3-[2-chloro 4-(3-thiényl acétsmido) phénoxy] 1,2-époxy propane préparé à l'exemple 29 et 10 ml d'isopropylamine on récupère 7 g de 1-[2-chloro 4-(3-thiényl acétamido) phénoxy] 3-isopro-pylamino 2-propanol sous forme de cristaux de point de fusion 132° C.

Chlorhydrate

Ces 7 g de cristaux sont dissous dans le méthanol et additionnés d'éther chlorhydrique jusqu'à pH acide. Après addition d'un peu d'éther les cristaux formés sont essorés, lavés avec un peu de méthanol et d'éther et séchés. On obtient 7,2 g du chlorhydrate de 1-[2-chloro 4-(3-thiényl acétamido) phénoxy] 3-isopropylamlio 2-propanol sous forme de cristaux de point de fusion 175-177° C.

**Exemple 47** 1-[2fluoro 4-(2-thiényl acétamido) phénoxy]

3-terbutylamino 2-propanol (base et maléate)

Formule (I) R = terbutyle X = F Y = H acétamide en 2

Selon le mode opérstoire de l'exemple 38 mais en utilisant 10 g de 3-[2-fluoro 4-(2-thiényl scétsmido) phénoxy] 1,2-époxy propane préparé à l'exemple 30 et 10 ml de terbutylamine on récupère 6,8 g de 1-[2-fluoro 4-(2-thiényl scétamido) phénoxy] 3-terbutylamino 2-propanol sous forme de cristaux de point de fusion 137° C.

Maléate

Ces 6,8 g de base sont dissous dans l'acétone et additionnés de 2,1 g d'acide maléique dissous dans l'acétone. Les cristaux formés sont essorés lavés à l'acétone et à l'éther puis séchés. On obtient 7,5 g du maléate de 1-[2-fluoro 4-(2-thiényl scétsmido) phénoxy] 3-terbutylsmino 2-propanol sous forme de cristaux de point de fusion 193-195° C.

**Exemple 48** 1-[2-fluoro 4-(2-thiényl acétamido) phénoxy]

3-isopropylamino 2-propanol (base et maléate)

Formule (I) R = isopropyle X = F Y = H acétamide en 2

Selon le mode opératoire de l'exemple 38 mais en utilisant 14 g de 3-[2-fluoro 4-(2-thiényl acétamido) phénoxy] 1,2-époxy propane préparé à l'exemple 30 et 20 ml d'isopropylamine on récupère 8,3 g de 1-(2-fluoro 4-(2-thiényl acétamido) phénoxy] 3-isopropylamino 2-propanol sous forme de cristaux de point de fusion 127° C.

Maléate

Ces 8,3 g de cristaux sont dissous dsns le méthanol et additionnés de la quantité stoéchiométrique d'acide maléique dissous dans le méthanol. Après addition d'éther les cristaux formés sont essorés, lavés avec un peu de méthanol et d'éther et séchés. On obtient 8 g du maléate de 1-[2-fluoro 4-(2-thiényl acétamido)phénoxy] 3-isopropylamino 2-propanol sous forme de cristaux de point de fusion 159-161° C.

**Exemple 49** 1-[2-fluoro 4-(3-thiényl acétamido) phénoxyl.

3-terbutylamino 2-propanol (base et maléate)

Formule (I) R = terbutyle X = F Y = H acétamide en 3

Selon le mode opératoire de 1 exemple 38 mais en utilisant 9,2 g de 3-[2-fluoro 4-(3-thiényl acétamido) phénoxy] 1,2-époxy propane préparé à l'exemple 31 et 10 ml de terbutylamine on récupère 8 g de 1-[2-fluoro 4-(3-thiényl acétamido)phénoxy]3-terbutylamino 2-propanol sous forme de cristaux de point de fusion 127° C.

Maléate

Ces 8 g de cristaux sont dissous dans l'acétone et additionnés de la quantité stoéchiodétrique d'acide maléique en solution dans l'acétone. Les cristaux formés sont essorés lavés à 1 acétone et à l'éther et séchés. On récupère 10 g de maléate de 1-[2-fluoro 4-(3-thiényl acétamido) phénoxy] 3-terbutylamino 2-propanol sous forme de cristaux de point de fusion 203-204° C.

**Exemple 50** 1-[2-fluoro 4-(3-thiényl acétamido phénoxy]

3-isopropylamino 2-propanol (base et chlorhydrate)

Formule (I) R = isopropyle X = F Y = H acétamide en 3

Selon le mode opératoire de l'exemple 38 mais en utilisant 9,2 g de 3-[2-fluoro 4-(3-thiényl acétsmido) phénoxy] 1,2-époxy propane préparé à l'exemple 31 et 10 ml d'isopropylamine on récupère 8,5 g de 1-[2-fluoro 4-(3-thiényl ,cétamido) phénoxy] 3-isopro-pylamino-2-propanol sous forme de cristaux de point de fusion 126° C.

Chlorhydrate

Ces 8,5 g de cristaux sont dissous dans l'acétone et additionnés d'éther chlorhydrique jusqu'à pH acide. Les cristaux formés sont essorés, lavés avec un peu d'acétone et d'éther et séchés. On récupère 8,8 g du chlorhydrate de 1-[2-fluoro 4-(3-thiényl acétamido) phénoxy] 3-isopropylsmino 2-propanol sous forme de cristaux ds point de fusion 173-175° C.

**Exemple 51** 1-12-méthoxy 4-(2-thiényl acétamido) phénoxy])

3-terbutylamino 2-propanol (maléate)

Formule (1) R = terbutyle X = $OCH_3$ Y = H acétamide en 2

Selon le mode opératoire de l'exemple 38 mais en utilisant 10 g de 3-[2-méthoxy 4-(2-thiényl acétamido) phénoxy] 1 2-époxy propane préparé à l'exemple 32 et 10 ml de terbutylamine on récupère 11 5 g de 1-[2-méthoxy 4-(2-thiényl acétamido) phénoxy] 3-terbutylamino 2-propanol sous forme d une huile. Cette huile est solubilisée dans 1 acétone et additionnée d une quantité stoéchiométrique d'acide maléique en solution dans l'acétone. Les cristaux qui se forment sont essorés lavés à l'acétone et à l'éther et séchés. On obtient 11 3 g de maléate de 1-[2-méthoxy 4-(2-thiényl acétamido) phénoxy] 3-terbutylamino 2-propanol sous forme de cristaux de point de fusion 156-159° C.

**Exemple 52** 1-(2-méthoxy 4-(2-thiényl acétamido phénoxy]

3-isopropylamino 2-propanol (base et maléate)

Formule (I) R = isopropyle X = $OCH_3$ Y = H acétamide en 2

Selon le mode opératoire de l'exemple 38 mais en utilisant 10 g de 3-[2-méthoxy 4-(2-thiényl acétamido) phénoxy] 1,2-époxy propane préparé à l'exemple 32 et 10 ml d isopropylamine on récupère 9 5 g de 1-[2-méthoxy 4-(2-thiényl acétsmido) phénoxy] 3-iso-propylamino 2-propanol sous forme de cristaux de point de fusion 140° C.

Maléate

Ces 9,5 g de cristaux sont dissous dans l'acétone et additionnés de la quantité stoéchiométrique d'acide maléique en solution dans l'acétone. Les cristaux qui se forment sont essorés lavés à l'acétone et à l'éther et séchés. On obtient 11 g de maléate de 1-[2-méthoxy 4-(2-thiényl acétamido) phénoxy] 3-isopropylamino 2-propanol sous forme de cristaux de point de fusion 115-117° C.

**Exemple 53** 1-[2-méthoxy 4-(3-thiényl acétamido) phénoxy]

3-terbutylamino 2-propanol (base et maléate).

Formule (I) R = terbutyle X = $OCH_3$ Y = H acétamide en 3

Selon le mode opératoire de l'exemple 38 mais en utilisant 11,2 g de 3-[2-méthoxy 4-(3-thiényl acétamido) phénoxy] 1,2-époxy propane préparé à l'exemple 33 et 15 ml de terbutylamine on récupère 10,7 g de 1-[2-méthoxy 4-(3-thiényl acétamido) phénoxy] 3-ter-butylamino 2-propanol sous forme de cristaux de point de fusion 97° C.

Maléate

Ces 10,7 g de cristaux sont dissous dans l'acétone et additionnés de la quantité stoéchiométrique d'acide maléique en solution dans l'acétone. Les cristaux formés sont essorés lavés à l'acétone et à l'éther et séchés. On obtient 12,5 g de maléate de 1-[2-méthoxy 4-(3-thiényl acétamido)phénoxy] 3-terbutylamino 2-propsnol sous forme de cristaux de point de fusion 152-155° C.

**Exemple 54** 1-[2-méthoxy 4-(3-thiényl acétamido) phénoxy]

3-isopropylamino 2-propanol

Formule (I) R = isopropyle X = $OCH_3$ Y = H acétamide en 3

Selon le mode opératoire de l'exemple 38 mais en utilisant 11,2 g de 3-[2-méthoxy 4-(3-thiényl acétamido) phénoxy] 1,2-époxy propane préparé à l'exemple 33 et 15 ml d'isopropylamine on récupère 7,8 g de 1-[2-méthoxy 4-(3-thiényl acétamido) phénoxy] 3-iso-propylamino 2-propanol sous forme de cristaux de point de fusion 141-143° C.

**Exemple 55** 1-[2 3-dichloro 4-(2-thiényl acétamido) phénoxy]

3-terbutylamino 2-propanol (base et maléate)

Formule (I) R = terbutyle X = Y = Cl acétamide en 2

Selon le mode opératoire de l'exemple 38 mais en utilisant 12 g de 3-[2 3-dichloro 4-(2-thiényl acétamido) phénoxy] 1,2-époxy propane préparé à l'exemple 34 et 15 ml de terbutylamine on récupère 9 g de 1-[2 3-dichloro 4-(2-thiényl acétamido) phénoxy] 3-terbutylamino 2-propanol sous forme de cristaux de point de fusion 98-100° C.

Maléste

Ces 9 g de cristaux sont dissous dans l'acétone et additionnés de la quantité stoéchiométrique d'acide maléique en solution dans l'acétone. Les cristaux formés sont essorés lavés à l'acétone et à l'éther et séchés. Après recristallisation dans un mélange acétate d'éthyle-acétone on récupère 5,7 g de maléste de 1-[2,3-dichloro 4-(2-thiényl acétamido) phénoxy] 3-terbutylamino 2-propanol sous forme de cristaux de point de fusion 128-130° C.

**Exemple 56** 1-[2 3-dichloro 4-(2-thiényl acétamido) phénoxy]

3-isopropylamino 2-propanol (base et chlorhydrate)

Formule (I) R = isopropyle X = Y = Cl acétamide en 2

Selon le mode opératoire de l'exemple 38 mais en utilisant 12 g de 3-[2 3-dichloro 4-(2-thiényl acétamido) phénoxy] 1,2-époxy propane préparé à l'exemple 34 et 15 ml d'isopropylamine on récupère 8,5 g de 1-[2 3-dichloro 4-(2-thiényl acétamido) phénoxy] 3-isopropylamino 2-propanol sous forme de cristaux de point de fusion 150-151° C.

Chlorhydrate

Ces 8,5 g de cristaux sont dissous dans l'acétone et additionnés d'éther chlorhydrique jusqu à pH acide. Les cristaux formés sont essorés lavés avec un peu d'acétone et d'éther et séchés durant 12 h à 100° C. On obtient 6,5 g du chlorhydrate de 1-[2 3-dichloro 4-(2-thiényl acétamido) phénoxy] 3-isopropylamino 2-props-nol sous forme de cristaux de point de fusion 125-128° C.

**Exemple 57** 1-[2 3-dichloro 4-(3-thiényl acétamido) phénoxy]

3-terbutylamino 2-propanol (base et maléate)

Formule (I) R = terbutyle X = Y = Cl acétamide en 3

Selon le mode opératoire de l'exemple 38 mais en utilisant 12 g de 3-[2 3-dichloro 4-(3-thiényl acétamido) phénoxy] 1 2-époxy propane préparé à l'exemple 35 et 15 ml de terbutylamine on récupère 10,4 g de 1-[2,3 dichloro 4-(3-thiénylacétamido) phénoxy] 3-terbutylamino 2-propanol sous forme de cristaux de point de fusion 96° C.

Maléate

Ces 10,4 g de cristaux sont dissous dans l'acétone et additionnés de la quantité stoéchiométrique d'acide malélque en solution dans l'acétone. Les cristaux formés sont essorés lavés à l'acétone et à l'éther et séchés. On récupère 11,4 g de maléate de 1-[2 3-dichloro 4-(3-thiényl acétamido) phénoxy] 3-terbutylamino 2-propanol sous forme de cristaux de point de fusion 133-135° C.

**Exemple 58** 1-[2,3-dichloro 4-(3-thiényl acétamido) phénoxy]

3-is-opropylamino 2-propanol (base et chlorhydrate)

Formule (I) R = isopropyle X = Y = Cl acétamide en 3

Selon le mode opératoire de l'exemple 38 mais en utilisant 12 g de 3-[2 3-dichloro 4-(3-thiényl acétamido) phénoxy] 1,2-époxy propane préparé à l'exemple 35 et 15 ml d'isopropylamine on récupère 10 g de 1-[2 3-dichloro 4-(3-thiényl acétamido) phénoxy] 3-iso-propylamino 2-propanol sous forme de cristaux de point de fusion 149° C.

Chlorhydrate

Ces 10 g de cristaux sont dissous dans 1 acétone et additionnés d'éther chlorhydrique jusqu'à pH acide. Les cristaux formés sont essorés lavés à l'acétone et l'éther et séchés. On récupère 10,3 g du chlorhydrate de 1-[2 3-dichloro 4-(3-thiényl acétamido) phénoxy] 3-isopropylamino 2-propanol sous forme de cristaux de point de fusion 169-171° C.

**Exemple 59** 1-[3-chloro 4-(2-thiényl acétamido)phénoxy]

3-terbutylamino 2-propanol (base et maléste)

Formule (I) R = terbutyle X = H Y = Cl acétamide en 2

Selon le mode opératoire de 1 exemple 38 mais en utilisant 10 g de 3-[3-chloro 4-(2-thiényl acétamido) phénoxy] 12-époxy propsne préparé à l'exemple 36 et 10 ml de terbutylamine on récupère 8,2 g de 1-[3-chloro 4-(2-thiényl acétamido) phénoxy] 3-terbutyl-amino 2-propanol sous forme de cristaux de point de fusion 100° C.

Maléate

Ces 8,2 g de cristaux sont dissous dans l'acétone et additionnés de la quantité stoéchiométrique d'acide maléique en solution dans l'acétone. Les cristaux formés sont essorés, lavés à l'acétone et à l'éther et séchés. On récupère 9,4 g de maléste de 1-[3-chloro 4-(2-thiényl acétamido) phénoxy] 3-terbutylamino 2-propanol sous forme de cristaux de point de fusion 164-167° C.

**Exemple 60** 1-44A3-chloro 4-(2-thiényl acétamido) phénoxy]

3-isopropylamino 2-propanol (base et maléate)

Formule (1) R = isopropyle X = H Y = Cl acétamide en 2

Selon le mode opératoire de l'éxemple 38 mais en utilisant 9 g de 3-[3-chloro 4-(2-thiényl acétamido) phénoxy] 1,2-époxy propane préparé à l'exemple 36 et 10 ml d'isopropylamine on récupère 8 g de 1-[3-chloro 4-(thiényl acétamido) phénoxy]3-isopropylamino 2-props-nol sous forme de cristaux de point de fusion 133° C.

Maléate

Ces 8 g de cristaux sont dissous dans l'acétone et additionés de la quantité stoéchiométrique d'acide maléique en solution dans l'acétone. Les cristaux formés sont essorés lavés à l'acétone et à l'éther et séchés. On récupère 7 8 g de maléate de 1-[3-chlo-ro 4-(2-thiényl acétamido) phénoxy] 3-isopropylamino 2-propanol sous forme de cristaux de point de fusion 103-108° C

**Exemple 61** 1-[3-chloro 4-(3-thiényl acétamido) phénoxy]

3-terbutylamino 2-propanol (base et maléate)

Formule (I) R = terbutyle X = H Y = Cl acétamide en 3

Selon le mode opératoire de l'exemple 38 mais en utilisant 12 g de 3-[3-chloro 4-(3-thiényl acétamido) phénoxy] 1,2-époxy propane préparé à l'exemple 37 et 15 ml de terbutylamine, on obtient 8,8 g de 1-[3-chloro 4-(3-thiényl acétsmido) phénoxy] 3-terbutylamino 2-propanol sous forme de cristaux de point de fusion 101° C.

Maléate

Ces 8,8 g de cristaux sont dissous dans l'acétone et additionnés de la quantité stoéchiométrique d'acide maléique en solution dans l'acétone. Les cristaux formés sont essorés lavés à l'acétone et à l'éther et séchés. On récupère 10 g de maléste de 1-[3-chloro 4-(3-thiényl acétamido) phénoxy] 3-terbutylamino 2-pro-panol sous forme de cristaux de point de fusion 163-165° C.

**Exemple 62** 1-[3-chloro 4-(3-thiényl acétamido) phénoxyl

3-isopropylamino 2-propanol (base et maléate)

Formule (I) R = isopropyle X = H Y = Cl acétamide en 3

Selon le mode opératoire de l'exemple 38 mais en utilisant 12 g de 3-[3-chloro 4-(3-thiényl acétamido) phénoxy] 1,2-époxy propane préparé à l'exemple 37 et 15 ml d'isopropylamine on obtient 8 g de 1-[3-chloro 4-(3-thiényl acétamido) phénoxy] 3-isopropyl-amino 2-propanol sous forme de cristaux de point de fusion 125° C.

Maléate

Ces 8 g de cristaux sont dissous dans l'acétone et additionnés de la quantité stoéchiométrique d'acide maléique en solution dans l'acétone. Les cristaux formés sont essorés lavés à l'acétone et à l'éther et séchés. On récupère 8 g de maléste de 1-[3-chloro 4-(3-thiényl acétamido) phénoxy] 3-isopropylsmino 2-propanol sous forme de cristaux de point de fusion 113-115° C.

**Activité β bloquante**

Methode

Des chiens tout-venants mâles ou femelles sont anesthésiés au mébubarbital sodique (30 mg/kg I.V.) et ventilés artificiellement par une pompe Pesty RPP. Ils sont supplémentés en oxygène.

On mesure:

- la pression carotidienne systolique et diastolique
- la force contractile du myocarde.

Les signaux sont amplifiés et enregistrés sur Dynograph Beckman.

L'activité β bloquante adrénergique a été étudiée vis-à-vis des effets $\beta_1$ (augmentation de la force contractile du myocarde) et des effets $\beta_2$ (diminution de la pression artérielle disstolique) de l'isoprénaline administrée par voie intraveineuse.

On calcule le pourcentsge d'inhibition des effets $\beta_1$ et des effets $\beta_2$ de l'isoprénaline en fonction des doses (mg.kg I.V.) des différents exemples.

Les exemples sont injectés dans le veine saphène aux doses (exprimées en base) de: 0,0003 0,001 0,003 0,01 0,1 1,0 et 4,0 ou 8,0 $mg.kg^{-1}$.

**Résultats**

Le tableau II ci-après résume les pourcentages d'inhibition des effets $\beta_1$ et $\beta_2$ de l'isoprénaline pour les différents exemples.

**Activité antihypertensive**

Méthode

Des rats mâles âgés de 13 à 21 semaines présentant une hypertension spontanée génétiquement transmissible (souche OKAMOTO provenance IFFA CREDO) sont placés dans une enceinte thermostatée à 38° C. Les rats sont laissés en situation libre. La pression artérielle est mesurée par un sphygmomanomètre Narco Biosystem. Un manchon est placé à la base de la queue et un détecteur enregistre le pouls en aval du manchon. Celui-ci est gonflé jusqu'à suppression du pouls il est ensuite progressivement dégonflé. La pression mesurée à la reprise des battements cardiaques est la pression artérielle systolique. On calcule la fréquence cardiaque à partir de l'enregistrement du pouls artériel. Les produits à étudiar sont mis en suspension et administrés par voie orale sous un volume de 1 ml pour 100 g de poids corporel. Les mesures sont répétées à intervalles réguliers.

**Résultats**

Les valeurs moyennes avant traitement pour les différents lots de rats sont pour la pression artérielle systolique de 190 à 200 mmHg et pour la fréquence cardiaque de 305 à 350 battements par minute.

Le tableau III ci-après donne la moyenne des variations de la pression systolique (mmHg) de la fréquence cardisque (battements/minute) et les écarts type à la moyenne obtenus 1 heure 3 heures et 5 heures après administration orale.

**Conclusion**

Les produits des différents exemples présentent une activité $B_1$ bloquante ils sont remarquables par leur sélectivité cardiaque par leur puissance d'activité et par leur activité antihypertensive après traitement oral en aigu chez le rat spontanément hypertendu.

Ils sont très peu toxiques après administration par voie intrapéritonéale ou par voie orale chez le rat.

**Tableau I**

5102-06 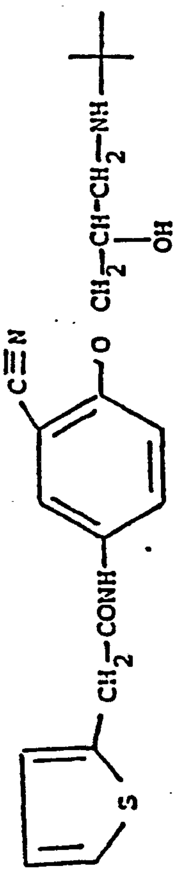 , HCl exemple 7 chlorhydrate

5102-07 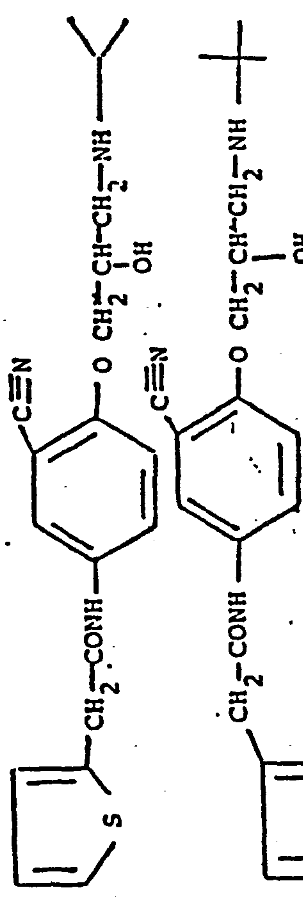 , HCl exemple 8 chlorhydrate

5102-17 , HCl exemple 9 chlorhydrate

5102-22 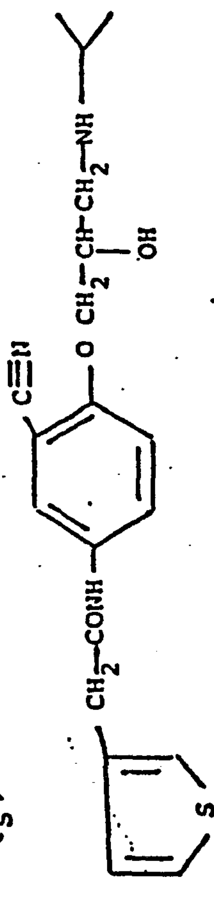 , HCl exemple 10 chlorhydrate

5103-04 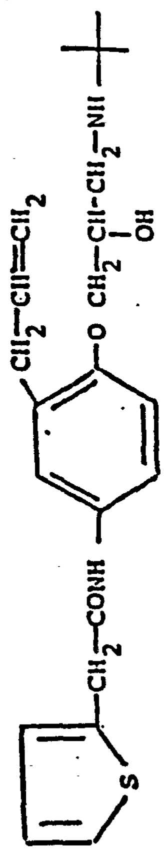 exemple 11

Tableau I (suite 1)

| | | |
|---|---|---|
| 5103-05 | 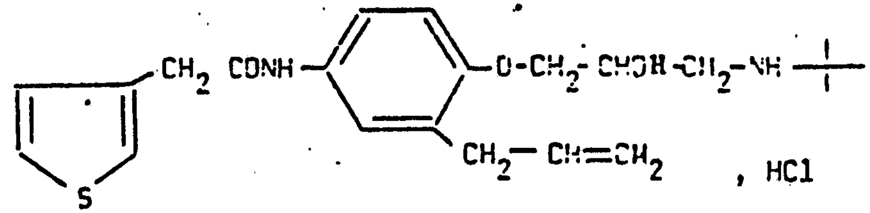 | exemple 38 |
| 5102-29 | 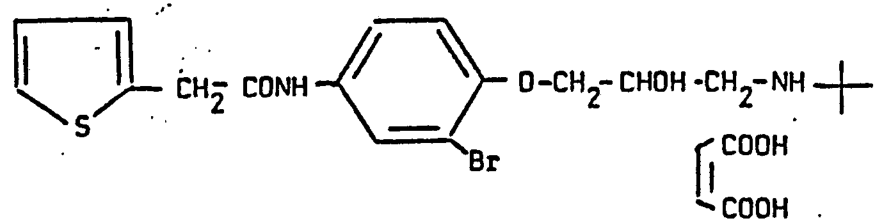 | exemple 39 |
| 5102-31 | 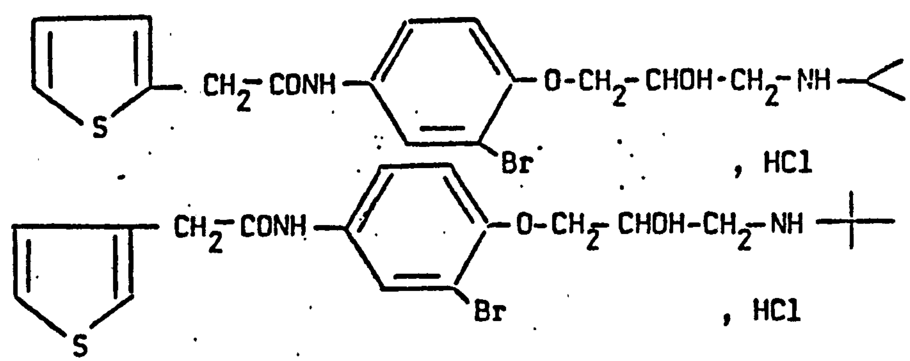 | exemple 40 |
| 5102-35 | | exemple 41 |
| 5102-37 | 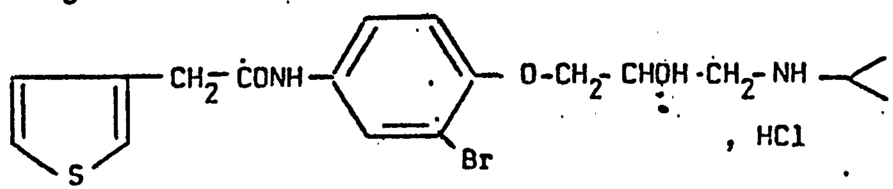 | exemple 42 |

**Tableau I** (suite 2)

| | | |
|---|---|---|
| exemple 43 | 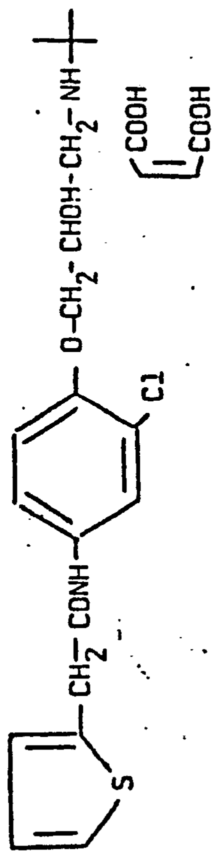 | 5102-30 |
| exemple 44 | 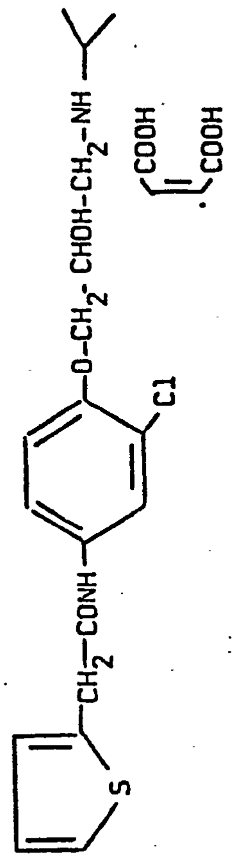 | 5102-33 |
| exemple 45 | 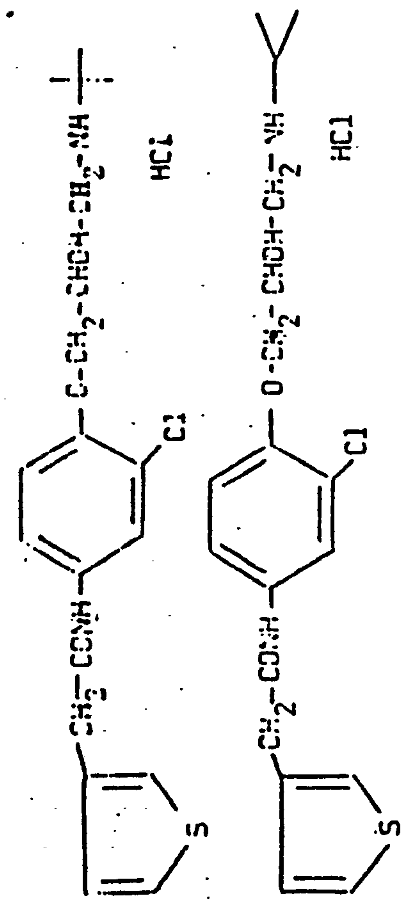 | 5102-36 |
| exemple 46 | | 5102-38 |

**Tableau I** (suite 3)

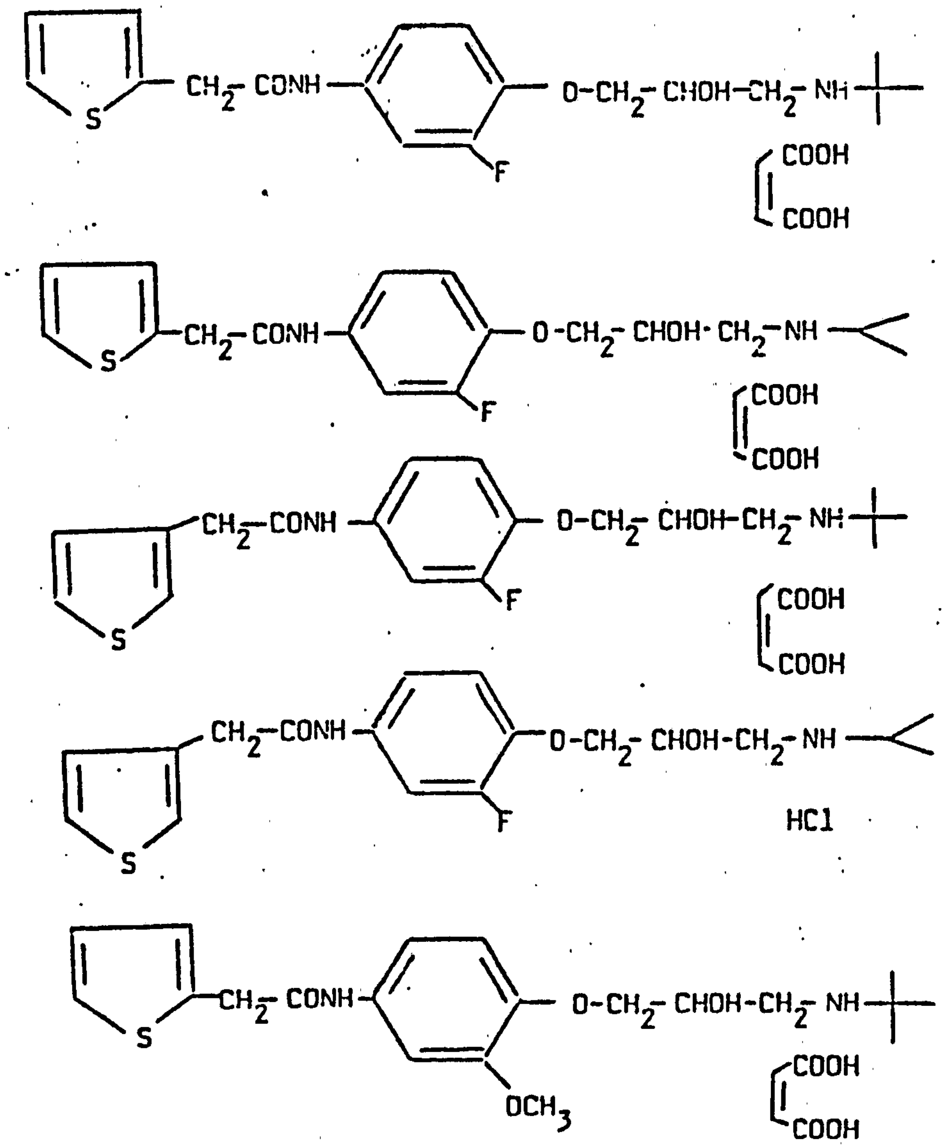

5102-41 — exemple 47

5102-40 — exemple 48

5102-49 — exemple 49

5102-47 — exemple 50

5102-52 — exemple 51

Tableau I (suite 4)

| | | |
|---|---|---|
| 5102-54 | 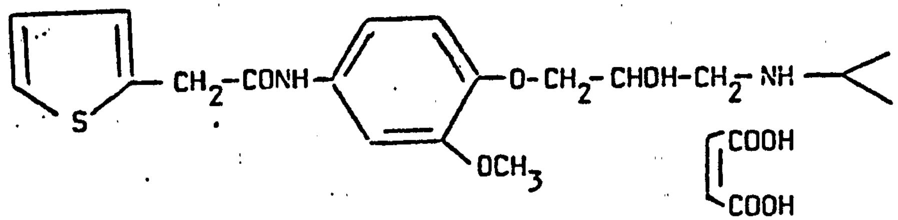 | exemple 52 |
| 5102-50 | 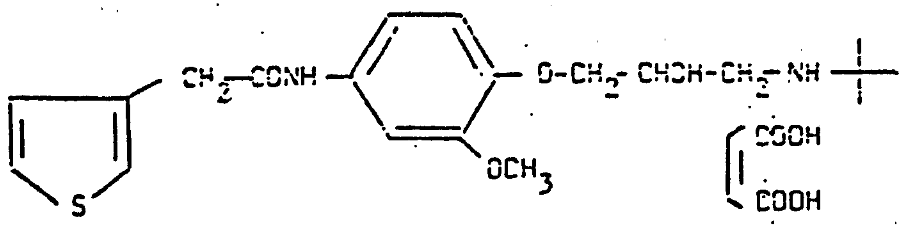 | exemple 53 |
| 5102-53 | 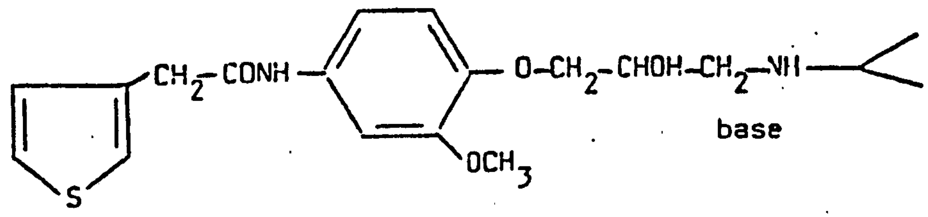 base | exemple 54 |
| 5102-58 | 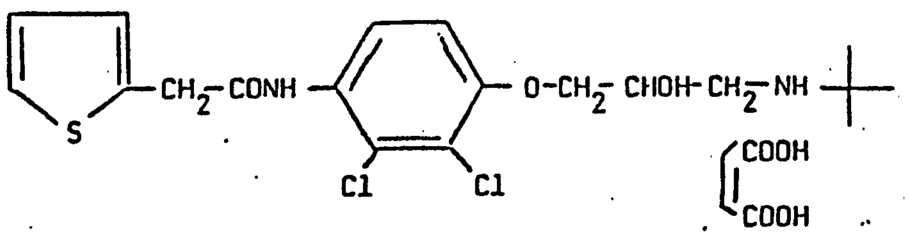 | exemple 55 |

Tableau I (suite 5)

exemple 56

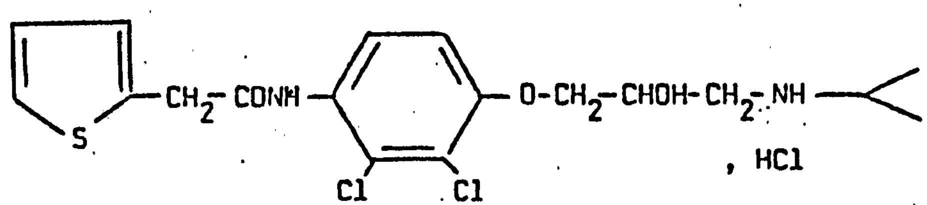

5102-61

exemple 57

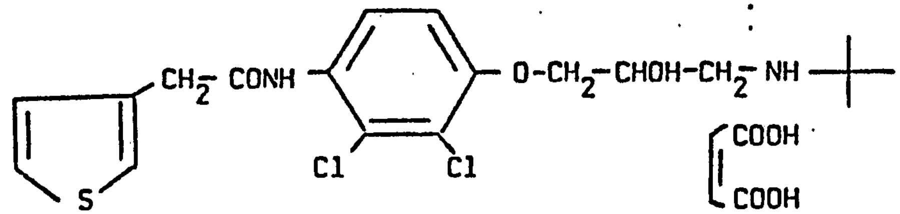

5102-55

exemple 58

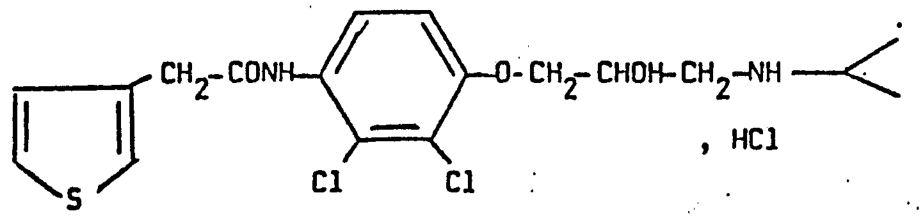

5102-56

exemple 59

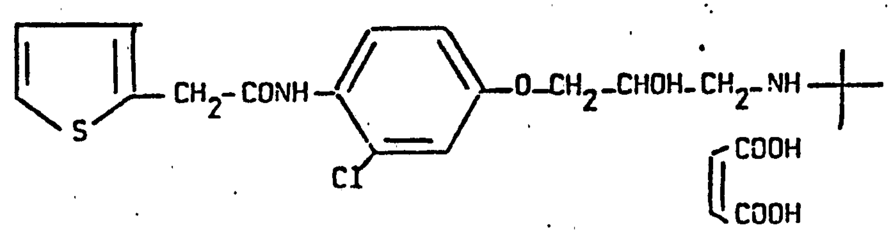

102-60

**Tableau I** (suite 6)

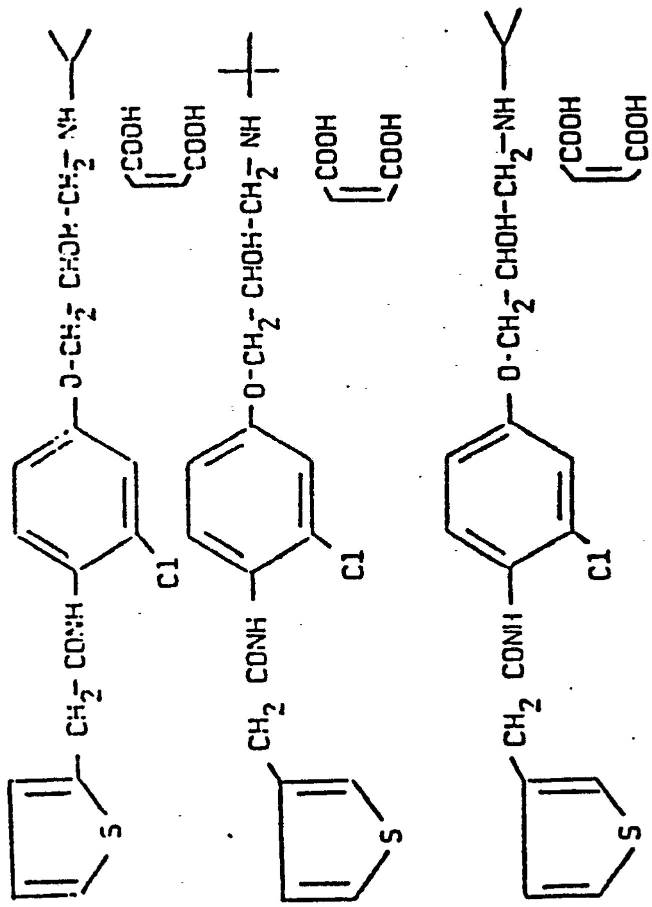

5102-62 — exemple 60

5102-57 — exemple 61

5102-59 — exemple 62

**Tableau II**

Pourcentages d'inhibition des effets $\beta_1$ de l'Isoprénaline

| EXEMPLES N° | Doses en mg.kg$^{-1}$ (voie intraveineuse) | | | | | | |
|---|---|---|---|---|---|---|---|
| | 0.001 | 0.003 | 0.01 | 0.1 | 1.0 | 4.0 | 8.0 |
| 7 chlorhydrate | 24 | 70 | 86 | 100 | 100 | | 100 |
| 8 chlorhydrate | 5 | 12 | 38 | 64 | 86 | 100 | |
| 9 chlorhydrate | 22 | 74 | 91 | 100 | 100 | | 100 |
| 11 | 11 | 37 | 70 | 100 | 100 | | 100 |

**Tableau II** (suite 1)

| Dose mg/kg I.V. | % d'inhibition des effets $\beta_1^{\oplus}$ de l'Isoprénaline | | | | | | |
|---|---|---|---|---|---|---|---|
| | 0,0003 | 0,001 | 0,003 | 0,010 | 0,100 | 1,00 | 8,00 |
| exemple 46 | -8 | -17 | -44 | -84 | -100 | -100 | -100 |
| exemple 45 | -12 | -30 | -60 | -86 | -100 | -100 | 100 |
| exemple 44 | -8 | -27 | -46 | -67 | -82 | -94 | -100 |
| exemple 43 | 0 | -18 | -49 | -82 | -100 | -100 | |
| exemple 42 | -13 | -29 | -51 | -70 | -100 | -100 | -100 |
| exemple 41 | | -12 | -49 | -83 | -97 | -100 | -100 |
| exemple 40 | 0 | 0 | -19 | -53 | -93 | -100 | -100 |
| exemple 39 | 0 | -11 | -65 | -89 | -100 | -97 | -96 |
| exemple 48 | -1 | -11 | -23 | -41 | -83 | -97 | -97 |
| exemple 47 | -5 | -12 | -33 | -66 | -96 | -100 | -100 |
| exemple 10 | -14 | -22 | | -47 | -91 | -95 | -100 |
| exemple 9 | | -22 | -74 | -91 | -100 | -100 | -100 |
| exemple 8 | | -5 | -12 | -38 | -64 | -86 | -100 |
| exemple 7 | | -24 | -70 | -86 | -100 | -100 | -100 |
| exemple 38 | 0 | -5 | -35 | -73 | -100 | -100 | -100 |
| exemple 11 | | -11 | -37 | -70 | -100 | -100 | -100 |

**Tableau II** (suite 2)

Pourcentages d'inhibition des effets $\beta_2$ de l'Isoprénaline

| EXEMPLES N° | Doses en $mg.kg^{-1}$ (voie intraveineuse) | | | | | | |
|---|---|---|---|---|---|---|---|
| | 0.001 | 0.003 | 0.01 | 0.1 | 1.0 | 4.0 | 8.0 |
| 7 Chlorhydrate | 0 | 4 | 16 | 36 | 88 | | 100 |
| 8 Chlorhydrate | 0 | 0 | 0 | 0 | 16 | 100 | |
| 9 Chlorhydrate | 0 | 0 | 8 | 42 | 100 | | 100 |
| 11 | 0 | 0 | 0 | 17 | 77 | | 100 |

**Tableau II** (suite 3)

| Dose mg/kg I.V. | % d'inhibition des effets $\beta_2^{\oplus}$ de l'Isoprénaline | | | | | | |
|---|---|---|---|---|---|---|---|
| | 0,0003 | 0,001 | 0,003 | 0,010 | 0,100 | 1,00 | 8,00 |
| exemple 46 | -5 | -5 | -5 | -20 | -19 | -62 | -100 |
| exemple 45 | -10 | -12 | -5 | -23 | -33 | -76 | -100 |
| exemple 44 | 0 | -11 | -22 | -18 | -15 | -42 | -100 |
| exemple 43 | 0 | 0 | 0 | 0 | -22 | -94 | |
| exemple 42 | 0 | 0 | 0 | 0 | 0 | -31 | -91 |
| exemple 41 | | -3 | 0 | -31 | -55 | -94 | -100 |
| exemple 40 | 0 | 0 | 0 | 0 | -4 | -50 | -93 |
| exemple 39 | 0 | 0 | 0 | -5 | -49 | -92 | -86 |
| exemple 48 | -17 | -27 | -31 | -46 | -52 | -68 | -90 |
| exemple 47 | -22 | -24 | -4 | 0 | -12 | -64 | -93 |
| exemple 10 | -8 | -21 | | -33 | -39 | -76 | -87 |
| exemple 9 | | 0 | 0 | -8 | -42 | -100 | -100, |
| exemple 8 | | 0 | 0 | 0 | 0 | -16 | -100 |
| exemple 7 | | 0 | -4 | -16 | -36 | -88 | -100 |
| exemple 38 | -6 | -4 | -4 | -6 | -27 | -77 | -100 |
| exemple 11 | | 0 | 0 | 0 | -17 | -77 | -100 |

**Tableau III**

Action antihypertensive chez le rat génétiquement hypertendu: Rat OKAMOTO

| EXEMPLES N° | Nombre de rats par dose | Doses $mg.kg^{-1}$ V.O | Pression systolique Δ (mmHg) | | | | | | Fréquence cardiaque Δ (battements/minute) | | | | | |
|---|---|---|---|---|---|---|---|---|---|---|---|---|---|---|
| | | | T+1 heure | | T+3 heures | | T+5 heures | | T+1 heure | | T + 3 heures | | T + 5 heures | |
| | | | m | (sm) | m | (sm) | m | (sm) | m | (sm) | m | (sm) | m | (sm) |
| 7 chlorhydrate | 11 | 128 | -27 | (4.5) | -27 | (4.8) | -31 | (4.9) | + 36 | (3.6) | +29 | (3.9) | + 25 | (7.8) |
| 8 chlorhydrate | 7 | 128 | -39 | (8.2) | -31 | (9.4) | -25 | (4.8) | + 31 | (10.8) | +36 | (6.9) | +12 | (12.8) |

**Tableau III** (suite 1)

| Produits | Nombre de rats par dose | Dose $mg.kg^{-1}$ V.O | Pression systolique Δ (mmHg) T+1 heure m (sm) | T+3 heures m (sm) | T+5 heures m (sm) | Fréquence cardiaque Δ (battements/min) T+1 heure m (sm) | T+3 heures m (sm) | T+5 heures m (sm) |
|---|---|---|---|---|---|---|---|---|
| 5102.38 exemple 46 | 12 | 8 | -21 (5) | | -12 (5) | +42 (7) | | +30 (6) |
| | 12 | 16 | -34 (6) | | -28 (7) | +49 (9) | | +28 (9) |
| | 12 | 32 | -37 (6) | | -36 (6) | +43 (8) | | +38 (7) |
| | 12 | 64 | -40 (7) | | -44 (5) | +72 (11) | | +52 (9) |
| 5102.36 exemple 45 | 6 | 64 | -32 (5) | -14 (9) | -28 (5) | +45 (6) | | +30 (10) |
| 5102.33 exemple 44 | 6 | 128 | -37 (11) | -48 (11) | -53 (11) | +68 (5) | +45 (12) | +52 (11) |
| 5102.30 exemple 43 | 6 | 64 | -29 (9) | -44 (6) | -44 (4) | +13 (20) | +23 (7) | +30 (12) |
| 5102.37 exemple 42 | 6 | 64 | -43 (8) | -55 (7) | -51 (10) | +50 (19) | +17 (12) | +33 (12) |
| 5102.35 exemple 41 | 6 | 64 | -44 (14) | -30 (10) | -33 (12) | +57 (18) | +55 (15) | +68 (16) |
| 5102.31 exemple 40 | 6 | 128 | -58 (10) | -63 (7) | -63 (3) | +23 (23) | +20 (14) | +18 (11) |
| 5102.29 exemple 39 | 6 | 128 | -33 (9) | -34 (11) | -43 (9) | +47 (15) | +56 (4) | +47 (4) |

Tableau III (suite 2)

| Produits | Nombre de rats par dose | Dose mg.kg$^{-1}$ V.O. | Pression systolique Δ (mmHg) | | | Fréquence cardiaque Δ (battements/min) | | |
|---|---|---|---|---|---|---|---|---|
| | | | T+1 heure m (sm) | T+3 heures m (sm) | T+5 heures m (sm) | T+1 heure m (sm) | T+3 heures m (sm) | T+5 heures m (sm) |
| 5102.40 exemple 48 | 6 | 64 | -51 (6) | -50 (6) | -45 (6) | +38 (10) | +41 (8) | +27 (10) |
| 5102.22 exemple 10 | 6 | 128 | -13 (12) | -5 (9) | -26 (8) | +25 (15) | +28 (7) | +25 (7) |
| 5102.17 exemple 9 | 6 | 64 | -16 (4) | -25 (8) | -38 (6) | +27 (11) | +13 (13) | +13 (13) |
| 5102.07 exemple 8 | 7 | 128 | -39 (8) | -31 (9) | -25 (5) | +31 (11) | +36 (7) | +12 (13) |
| 5102.06 exemple 7 | 12 | 128 | -27 (5) | -27 (5) | -31 (5) | +36 (4) | +29 (4) | +25 (9) |
| 5103.05 exemple 38 | 6 | 64 | -31 (5) | -26 (5) | -34 (7) | +39 (9) | +21 (9) | +29 (13) |
| 5103.04 exemple 11 | 6 | 64 | -54 (1) | -49 (10) | -44 (11) | +7 (8) | +10 (9) | 0 (9) |

m = moyenne

(sm) = écart type à la moyenne

**Revendications**

1. Composés caractérisés en ce qu'ils répondent à la formule générale:

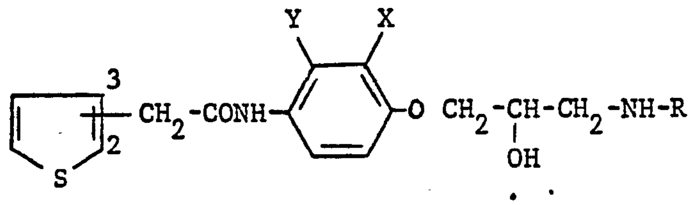

dans laquelle
- la fonction acétamide peut être en position -2 ou -3 sur l'hétérocycle thiophène
- X représente un atome d'halogène, un groupement allylique ou méthoxy ou un groupement nitrile, et peut être l'hydrogène dans le cas seulement où Y est un halogène,
- Y représente généralement l'hydrogène mais peut être un halogène
- R représente un radical alkyle inférieur en $C_1$-$C_6$ linéaire ou ramifié

et leurs sels d'addition d'acides.

2. Composés selon la revendication 1, caractérisés en ce que R représente le radical terbutyle ou isopropyle.

3. Procédé de préparstion des composés de formule (I) selon la revendication 1 ou 2 caractérisé en ce que l'on fait réagir une base de formule $NH_2R$, R étant tel que défini dans la revendication 1 ou 2 sur un composé de formule (II):

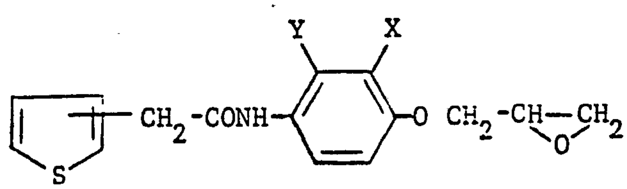

(II)

dans laquelle X et Y sont tels que définis dans la revendication 1 ou 2, sans solvant ou dans un solvant organique usuel, à une température comprise entre 20 et 150° C.

4. Procédé selon la revendication 3 caractérisé en ce que l'on fait réagir un phénol de formule (III):

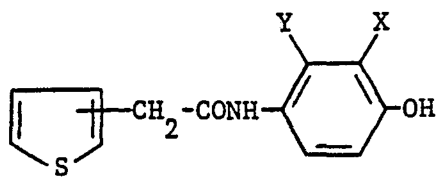

(III)

dans laquelle X et Y sont tels que définis dans la revendication 1 ou 2, pour obtenir le composé de formule II avec un excès d'épichlorhydrine en présence comme catalyseur du benzyl triméthyl ammonium chlorure à une température de 110 à 130° C.

5. Procédé selon la revendication 4 caractérisé en ce que le composé de formule (III) est préparé par réaction du chlorure de l'acide thiényl acétique correspondant sur des amino phénols de formule (IV):

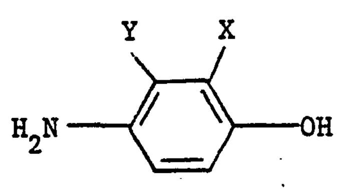

dans laquelle X et Y sont tels que définis dans la revendication 1 ou 2 soit en présence du double d'amino phénol soit en présence d'une base la triéthylamine dans un solvant usuel.

6. Composés caractérisés en ce qu'ils répondent à la formule:

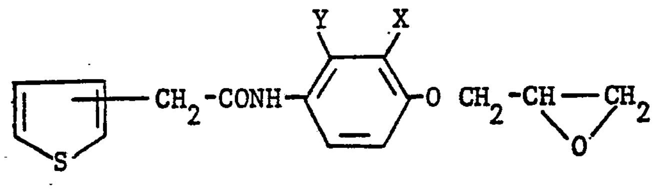

(II)

dans laquelle X représente un atome d'halogène un groupement allylique ou un groupement nitrile et Y représente généralement l'hydrogène mais peut être un halogène

7. Composés caractérisés en ce qu'ils répondent à la formule:

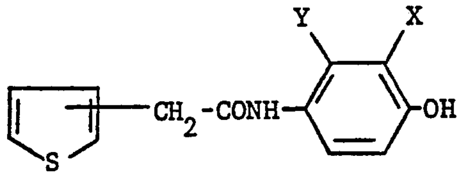

(III)

dans laquelle X représente un atome d'halogène un groupement allylique ou un groupement nitrile, et Y représente généralement l'hydrogène mais peut être un halogène.

8. Médicaments utiles notamment comme β-bloqueurs antihypertenseurs caractérisés en ce qu'ils contiennent au moins un composé selon l'une quelconque des revendications 1 ou 2.

**Patentansprüche**

1. Verbindungen, dadurch gekennzeichnet, daß sie der allgemeinen Formel

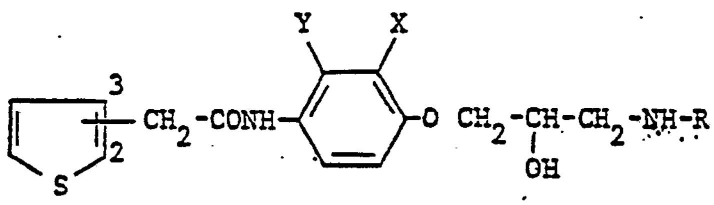

entsprechen, worin

- die Acetamidfunktion in der Position 2 oder 3 des Thiophen-Heterocyclus' stehen kann,
- X ein Halogenatom, eine Allyl- oder Methoxygruppe oder eine Nitrilgruppe darstellt und für den einzigen Fall, daß Y ein Halogen ist, Wasserstoff sein kann,
- Y im allgemeinen für Wasserstoff steht, aber auch ein Halogen sein kann,
- R einen geraden oder verzweigten $C_1$-$C_6$-Niedrigalkyl-rest darstellt,

und ihre Säureadditionssalze.

2. Verbindungen nach Anspruch 1, dadurch gekennzeichnet, daß R die Gruppe tert.Butyl oder Isopropyl darstellt.

3. Verfahren zur Herstellung der Verbindungen der Formel (I) nach Anspruch 1 oder 2, dadurch gekennzeichnet, daß man eine Base der Formel $NH_2R$, worin R die in Anspruch 1 oder 2 angegebene Bedeutung hat, mit einer Verbindung der Formel (II)

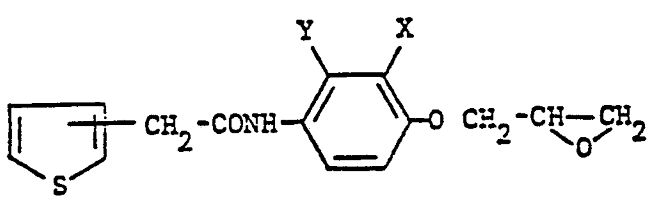

(II)

worin X und Y die in Anspruch 1 oder 2 angegebene Bedeutung haben, ohne Lösungsmittel oder in einem herkömmlichen organischen Lösungsmittel bei einer Temperatur zwischen 20 und 150° zur Umsetzung bringt.

4. Verfahren nach Anspruch 3, dadurch gekennzeichnet, daß man ein Phenol der Formel (III)

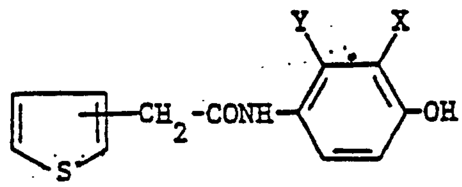

(III)

worin X und Y die in Anspruch 1 oder 2 angegebene Bedeutung haben, zur Gewinnung der Verbindung der Formel II mit einem Überschuß an Epichlorhydrin in Gegenwart von Benzyltrimethylammoniumchlorid als Katalysator bei einer Temperatur von 110 bis 130° zur Umsetzung bringt.

5. Verfahren nach Anspruch 4, dadurch gekennzeichnet, daß die Verbindung der Formel (III) hergestellt wird durch Umsetzen des entsprechenden Thienylessigsäurechlorids mit Aminophenolen der Formel (IV)

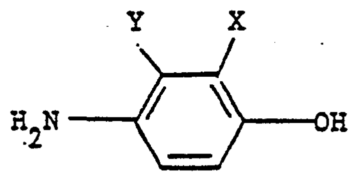

worin X und Y die in Anspruch 1 oder 2 angegebene Bedeutung haben, entweder in Gegenwart des Doppelten an Aminophenol oder in Gegenwart einer Base, nämlich Triäthylamin, in einem herkömmlichen Lösungsmittel.

6. Verbindungen, dadurch gekennzeichnet, daß sie der Formel

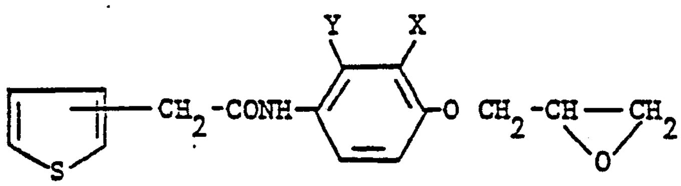

(II)

entsprechen, worin X ein Halogenatom, eine Allylgruppe oder eine Nitrilgruppe darstellt und Y im allgemeinen für Wasserstoff steht, aber auch ein Halogen sein kann.

7. Verbindungen, dadurch gekennzeichnet, daß sie der Formel

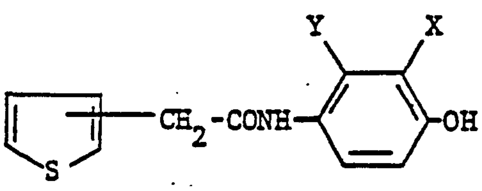

(III)

entsprechen, worin X ein Halogenatom, eine Allylgruppe oder eine Nitrilgruppe darstellt und Y im allgemeinen für Wasserstoff steht, aber auch ein Halogen sein kann.

8. Medikamente, die insbesondere als antihypertensive β-Blocker nützlich sind, dadurch gekennzeichnet, daß sie mindestens eine Verbindung nach einem der Ansprüche 1 oder 2 enthalten.

## Claims

1. Compounds characterized in that they respond to the general formula:

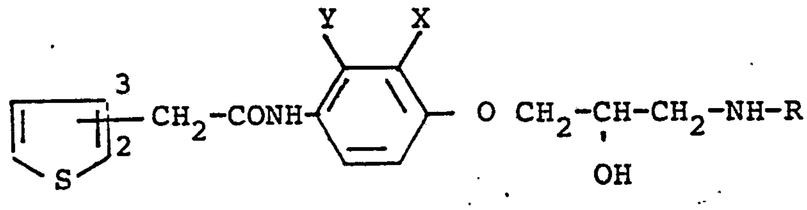

in which
- the acetamide function may be in -2 or -3 position over the thiophene heterocycle,
- X is a halogen atom, an allyl, methoxy or nitrile group, and may be hydrogen only in the case where Y is a halogen,
- Y is generally hydrogen but may be a halogen,
- R is a straight or branched lower alkyl radical in $C_1$-$C_6$,

and their acid addition salts.

2. Compounds according to claim 1, characterized in that R is the terbutyl or isopropyl radical.

3. Process for preparing compounds of formula (1) according to claim or 2, characterized in that it consists in reacting a base of formula $NH_2R_1$, R being such as defined in claim 1 or 2, over a compound of formula (II);

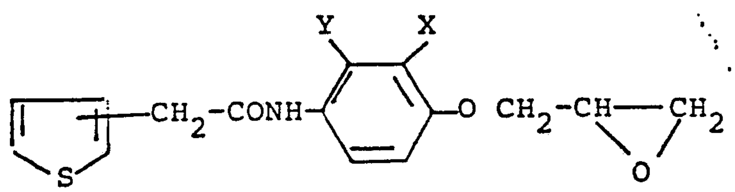

(II)

in which X and Y are such as defined in claim 1 or 2, without solvent or in a conventional organic solvent, at a temperature between 20 and 150° C.

4. Process according to claim 3, characterized in that a phenol of formula (III):

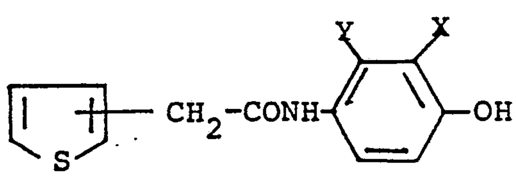

(III)

in which X and Y are such as defined in claim 1 or 2, is reacted to obtain the compound of formula II with an excess of epichlorohydrin in the presence of benzyl trimethyl ammonium chloride as catalyst at a temperature of 110 to 130° C.

5. Process according to claim 4, characterized in that the compound of formula III is prepared by reacting the chloride of the corresponding acetic thienyl acid over amino phenols of formula IV:

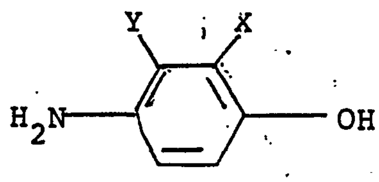

in which X and Y are such as defined in claim 1 or 2, either in the presence of double amino phenol, or in the presence of a base, the triethylamine, in a conventional solvent.

6. Compounds characterized in that they respond to the formula:

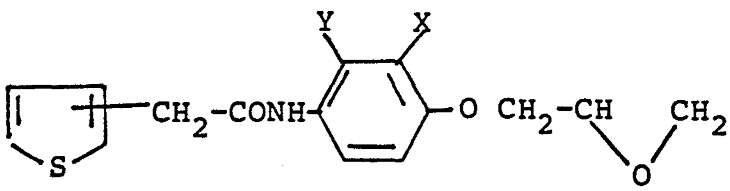

(II)

in which X is a halogen atom, an allyl or nitrile group, and Y generally is hydrogen but may be a halogen.

7. Compounds characterized in that they respond to the formula:

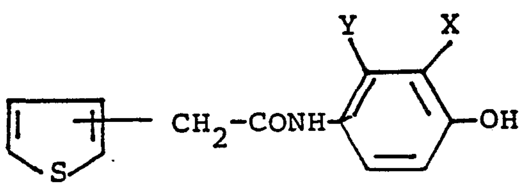

(III)

in which X is a halogen atom, an allyl or nitrile group, and Y generally is hydrogen but may be a halogen.

8. Drugs useful particularly as ß-blocking antihypertensive agent, characterized in that they contain at least one compound according to any one of claim or 2.